(19) Europäisches Patentamt
European Patent Office
Office européen des brevets

(11) EP 4 340 842 B1

(12) # EUROPEAN PATENT SPECIFICATION

(45) Date of publication and mention of the grant of the patent:
**27.08.2025 Bulletin 2025/35**

(21) Application number: **22729925.2**

(22) Date of filing: **17.05.2022**

(51) International Patent Classification (IPC):
***A61K 31/519*** (2006.01) ***A61P 35/00*** (2006.01)
***A61P 35/04*** (2006.01)

(52) Cooperative Patent Classification (CPC):
**A61K 31/519; A61P 35/00; A61P 35/04**

(86) International application number:
**PCT/US2022/029677**

(87) International publication number:
**WO 2022/245857 (24.11.2022 Gazette 2022/47)**

(54) **SOTORASIB FOR USE IN THE TREATMENT OF CANCER**
SOTORASIB ZUR VERWENDUNG BEI DER BEHANDLUNG VON KREBS
SOTORASIB POUR UTILISATION DANS LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.05.2021 US 202163190061 P**

(43) Date of publication of application:
**27.03.2024 Bulletin 2024/13**

(73) Proprietor: **Amgen Inc.**
**Thousand Oaks, California 91320 (US)**

(72) Inventors:
- **HENARY, Haby**
  **Thousand Oaks, California 91320-1799 (US)**
- **NGARMCHAMNANRITH, Gataree**
  **Thousand Oaks, California 91320-1799 (US)**
- **PARK, Joseph**
  **Thousand Oaks, California 91320-1799 (US)**
- **DUTTA, Sandeep**
  **Thousand Oaks, California 91320-1799 (US)**
- **FRIBERG, Gregory**
  **Thousand Oaks, California 91320-1799 (US)**
- **HOUK, Brett E.**
  **Thousand Oaks, California 91320-1799 (US)**
- **MATHER, Omar**
  **Thousand Oaks, California 91320-1799 (US)**

(74) Representative: **Murgitroyd & Company**
**165-169 Scotland Street**
**Glasgow G5 8PL (GB)**

(56) References cited:
- **HONG DAVID S. ET AL: "KRAS G12C Inhibition with Sotorasib in Advanced Solid Tumors", THE NEW ENGLAND JOURNAL OF MEDICINE, vol. 383, no. 13, 24 September 2020 (2020-09-24), US, pages 1207 - 1217, XP055911440, ISSN: 0028-4793, Retrieved from the Internet <URL:https://www.nejm.org/doi/pdf/10.1056/NEJMoa1917239?articleTools=true> DOI: 10.1056/NEJMoa1917239**
- **CANON JUDE ET AL: "The clinical KRAS(G12C) inhibitor AMG 510 drives anti-tumour immunity", NATURE, NATURE PUBLISHING GROUP UK, LONDON, vol. 575, no. 7781, 30 October 2019 (2019-10-30), pages 217 - 223, XP036920971, ISSN: 0028-0836, [retrieved on 20191030], DOI: 10.1038/S41586-019-1694-1**
- **SCHIRRIPA MARTA ET AL: "KRAS G12C Metastatic Colorectal Cancer: Specific Features of a New Emerging Target Population", CLINICAL COLORECTAL CANCER UNITED STATES 09 2020, CLINICAL COLORECTAL CANCER UNITED STATES 09 2020, CLINICAL COLORECTAL CANCER UNITED STATES 09 2020, vol. 19, no. 3, 31 August 2020 (2020-08-31), pages 219 - 225, XP009537542, ISSN: 1938-0674, DOI: 10.1016/J.CLCC.2020.04.009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

### CROSS-REFERENCE TO RELATED APPLICATIONS

This application claims the benefit of United States Provisional Application No. 63/190,061, filed May 18, 2021.

### BACKGROUND

The rat sarcoma (RAS) proto-oncogene has been identified as an oncogenic driver of tumorigenesis in cancers, such as non-small cell lung cancer (NSCLC) and colorectal cancer (CRC). The RAS family consists of 3 closely related genes that express guanosine triphosphate (GTP)-ases responsible for regulating cellular proliferation and survival. The RAS proteins, Kirsten rat sarcoma viral oncogene homolog (KRAS), Harvey rat sarcoma viral oncogene homolog (HRAS), and neuroblastoma RAS viral oncogene homolog (NRAS) can be mutationally activated at codons 12, 13, or 61, leading to human cancers. Different tumor types are associated with mutations in certain isoforms of RAS, with KRAS being the most frequently mutated isoform in most cancers. While the role of KRAS mutations in human cancers has been known for decades, no anti-cancer therapies specifically targeting KRAS mutations have been successfully developed, until recently, largely because the protein had been considered intractable for inhibition by small molecules. Hong David et al., N Engl J Med, 2020 Sep 24;383(13):1207-1217, disclosed that no therapies for targeting KRAS mutations in cancer were approved. The *KRAS* p.G12C mutation occurs in 13% of non-small-cell lung cancers (NSCLCs) and in 1 to 3% of colorectal cancers and other cancers. Sotorasib is a small molecule that selectively and irreversibly targets $KRAS^{G12C}$.

### SUMMARY

Provided herein are methods of treating cancer comprising a *KRAS G12C* mutation in a patient with active brain metastases, comprising administering sotorasib orally to the patient in an amount effective to treat cancer. The references to methods of treatment in the subsequent paragraphs are to be interpreted as references to the compounds, pharmaceutical compositions and medicaments of the present invention for use in a method for treatment of the human (or animal) body by therapy.

In various embodiments, the sotorasib is administered once per day. The sotorasib is administered orally. In various embodiments, the patient is administered sotorasib for at least one month. In various embodiments, the patient is administered sotorasib for at least three months. In various embodiments, the patient is administered sotorasib for at least six months.

In various embodiments, the cancer is a solid tumor. In various embodiments, the cancer is non-small cell lung cancer. In various embodiments, the cancer is colorectal cancer. In various embodiments, the cancer is pancreatic cancer. In various embodiments, the cancer is small bowel cancer, appendiceal cancer, endometrial cancer, hepatobiliary cancer, small cell lung cancer, cervical cancer, germ cell tumor, ovarian cancer, gastrointestinal neuroendocrine tumor, bladder cancer, myelodysplastic/myeloproliferative neoplasms, head and neck cancer, esophagogastric cancer, soft tissue sarcoma, mesothelioma, thyroid cancer, leukemia, or melanoma.

### BRIEF DESCRIPTION OF DRAWINGS

Figure 1 shows tumor shrinkage in brain lesions by subject (waterfall plot of RANO-BM best shrinkage per investigator).

Figure 2 shows the mean plasma concentration time profile after once daily oral administration of 180, 360, 720, or 960 mg sotorasib on Day 1, where N indicates number of observations across data points.

Figure 3 shows the mean plasma concentration time profile after once daily oral administration of 180, 360, 720, or 960 mg sotorasib on Day 8, where N indicates number of observations across data points.

### DETAILED DESCRIPTION

Provided herein are methods of treating a cancer comprising a *KRAS* G12C mutation in a patient with active brain metastases, comprising administering sotorasib orally to the patient in an amount effective to treat the cancer. The invention is defined by the claims and any other aspects, configurations or embodiments set forth herein not falling within the scope of the claims are for information only.

The phrase "active brain metastases" as used herein refers to a cancer that has spread from the original (primary, non-brain) tumor to the brain. Active brain metastases can be assessed by the presence of intracranial lesions. It is to be understood that while "metastases" is plural, patients exhibiting only one intracranial lesion under the criteria noted below is a patient who has "active brain metastases." In some embodiments, a patient having active brain metastases has at least one measurable intracranial lesion > 5 mm. In some embodiments, a patient having active brain metastases has at least one measurable intracranial lesion > 5 mm but < 10 mm. In some embodiments, a patient having active brain metastases has at least one measurable intracranial lesion > 10 mm. A patient is not considered a patient with active brain metastases if the patient has had one or more intracranial lesions resected or has received radiation therapy prior to the first administration of sotorasib (e.g., 4 week prior to the first administration of sotorasib) and said patient meets all of the following criteria: (a) residual neurological symptoms attributable to an intracranial lesion of a grade ≤ 2; (b) on stable doses of dexamethasone, if applicable; and (c) follow-up magnetic resonance imaging (MRI) performed within 30 days shows no new intracranial lesions appearing or growth of existing intracranial lesions. For determining the grade of any neurological symptom attributable to an intracranial lesion, see National Cancer Institute Common Terminology Criteria for Adverse Events v5.0 (NCI CTCAE) published Nov. 27, 2017 by the National Cancer Institute.

Sotorasib is a small molecule that irreversibly inhibits the $KRAS^{G12C}$ mutant protein. Sotorasib is also referred to as AMG 510 or 6-fluoro-7-(2-fluoro-6-hydroxyphenyl)-(1*M*)-1-[4-methyl-2-(propan-2-yl)pyridin-3-yl]-4-[(2*S*)-2-methyl-4-(prop-2-enoyl)piperazin-1-yl)pyrido[2,3-*d*]pyrimidin-2(1*H*)-one and has the following structure:

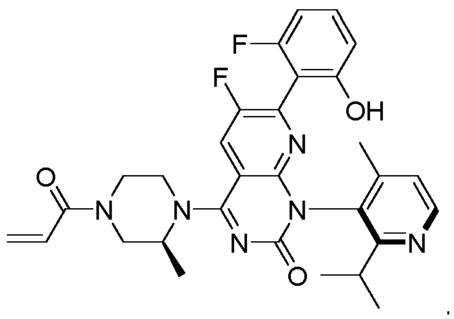

Sotorasib binds to the P2 pocket of KRAS adjacent to the mutant cysteine at position 12 and the nucleotide-binding pocket. The inhibitor contains a thiol reactive portion which covalently modifies the cysteine residue and locks $KRAS^{G12C}$ in an inactive, guanosine diphosphate (GDP) bound conformation. This blocks the interaction of KRAS with effectors such as rapidly accelerated fibrosarcoma (RAF), thereby preventing downstream signaling, including the phosphorylation of extracellular signal regulated kinase (ERK) (Cully and Downward, 2008; Ostrem et al., 2013; Simanshu et al., 2017). Inactivation of KRAS by RNA interference (RNAi) or small molecule inhibition has previously demonstrated an inhibition of cell growth and induction of apoptosis in tumor cell lines and xenografts harboring KRAS mutations (including the *KRAS* G12C mutation) (Janes et al., 2018; McDonald et al., 2017; Xie et al., 2017; Ostrem and Shokat, 2016; Patricelli et al., 2016). Studies with sotorasib have confirmed these *in vitro* findings and have likewise demonstrated inhibition of growth and regression of cells and tumors harboring *KRAS G12C* mutations (Canon et al., 2019).

Sotorasib can be administered to the patient in an amount ranging from 240 mg to 960 mg. In some embodiments, the methods comprise administering 960 mg sotorasib to the patient once daily. In some embodiments, the methods comprise administering 480 mg sotorasib to the patient once daily. In some embodiments, the methods comprise administering 240 mg to the patient once daily. In some embodiments, the methods comprise administering 480 mg to the patient twice daily. In some embodiments, the methods comprise administering 240 mg to the patient twice daily.

In various embodiments, the sotorasib is administered orally. In various embodiments, the sotorasib is administered with food. In various embodiments, the sotorasib is administered without food.

In various embodiments, the patient is further in need of treatment with an acid-reducing agent. Acid-reducing agents include, but are not limited to a proton pump inhibitor (PPI), a H2 receptor antagonist (H2RA), and a locally acting antacid. In one embodiment, the patient is further in need of treatment with a PPI or a H2RA. Exemplary PPIs include, but are not limited to, omeprazole, pantoprazole, esomeprazole, lansoprazole, rabeprazole, and dexlansoprazole. Exemplary H2RAs include, but are not limited to, famotidine, ranitidine, cimetidine, nizatidine, roxatidine and lafutidine. Exemplary locally acting antacids include, but are not limited to, sodium bicarbonate, calcium carbonate, aluminum hydroxide, and magnesium hydroxide. In some embodiments, the patient who is in further need of treatment with an acid reducing agent is not administered a proton pump inhibitor or a H2 receptor antagonist in combination with sotorasib. In some embodiments, sotorasib is administered about 4 hours before or about 10 hours after a locally acting antacid.

In various embodiments, the patient is in further need of treatment with a CYP3A4 inducer. In some embodiments, the patient is not administered a CYP3A4 inducer in combination with sotorasib. Exemplary CYP3A4 inducers include, but are not limited to, barbiturates, brigatinib, carbamazepine, clobazam, dabrafenib, efavirenz, elagolix,

enzalutamide, eslicarbazepine, glucocorticoids, letermovir, lorlatinib, modafinil, nevirapine, oritavancin, oxcarbazepine, perampanel, phenobarbital, phenytoin, pioglitazone, rifabutin, rifampin, telotristat, and troglitazone. See, e.g., Flockhart DA, Drug Interactions: Cytochrome P450 Drug Interaction Table. Indiana University School of Medicine (2007), www.drug-interactions.medicine.iu.edu, accessed May 2021. In some embodiments, the patient is not administered a strong CYP3A4 inducer in combination with sotorasib. Exemplary strong CYP3A4 inducers include, but are not limited to, phenytoin and rifampin. See, e.g., www.fda.gov/drugs/drug-interactions-labeling/drug-development-and-drug-interactions-table-substrates-inhibitors-and-inducers, accessed May 2021.

In various embodiments, the patient is in further need of treatment with a CYP3A4 substrate. In some embodiments, the patient is not administered a CYP3A4 substrate in combination with sotorasib. Exemplary CYP3A4 substrates include, but are not limited to, abemaciclib, abiraterone, acalabrutinib, alectinib, alfentanil, alprazolam, amitriptyline, amlodipine, apixaban, aprepitant, aripiprazole, astemizole, atorvastatin, avanafil, axitinib, boceprevir, bosutinib, brexpiprazole, brigatinib, buspirone, cafergot, caffeine, carbamazepine, cariprazine, ceritinib, cerivastatin, chlorpheniramine, cilostazol, cisapride, citalopram, clarithromycin, clobazam, clopidogrel, cobimetinib, cocaine, codeine, colchicine, copanlisib, crizotinib, cyclosporine, dabrafenib, daclatasvir, dapsone, deflazacort, dexamethasone, dextromethorphan, diazepam, diltiazem, docetaxel, dolutegravir, domperidone, doxepin, elagolix, elbasvir/grazoprevir, eliglustat, enzalutamide, eplerenone, erythromycin, escitalopram, esomeprazole, estradiol, felodipine, fentanyl, finasteride, flibanserin, gleevec, haloperidol, hydrocortisone, ibrutinib, idelalisib, indacaterol, indinavir, irinotecan, isavuconazonium, ivabradine, ivacaftor, lansoprazole, lenvatinib, lercanidipine, lidocaine, linagliptin, lovastatin, macitentan, methadone, midazolam, naldemedine, naloxegol, nateglinide, nelfinavir, neratinib, netupitant/palonosetron, nevirapine, nifedipine, nisoldipine, nitrendipine, olaparib, omeprazole, ondansetron, osimertinib, ospemifene, palbociclib, panobinostat, pantoprazole, perampanel, pimavanserin, pimozide, pomalidomide, ponatinib, progesterone, propranolol, quetiapine, quinidine, quinine, regorafenib, ribociclib, rilpivirine, risperidone, ritonavir, rivaroxaban, roflumilast, rolapitant, romidepsin, ruxolitinib, salmeterol, saquinavir, selexipag, sildenafil, simeprevir, simvastatin, sirolimus, sonidegib, sorafenib, sunitinib, suvorexant, tacrolimus(fk506), tamoxifen, tasimelteon, taxol, telaprevir, telithromycin, terfenadine, testosterone, ticagrelor, tofacitinib, tolvaptan, torisel, tramadol, trazodone, valbenazine, vandetanib, velpatasvir, vemurafenib, venetoclax, venlafaxine, verapamil, vilazodone, vincristine, vorapaxar, voriconazole, zaleplon, and ziprasidone. See, e.g., Flockhart DA, Drug Interactions: Cytochrome P450 Drug Interaction Table. Indiana University School of Medicine (2007), https://drug-interactions.medicine.iu.edu, accessed May 2021.

In various embodiments, the patient is in further need of treatment with a P-glycoprotein (P-gp) substrate. In some embodiments, the patient is not administered a P-gp substrate in combination with sotorasib. Exemplary P-gp substrates include, but are not limited to dabigatran etexilate, digoxin, fexofenadine, everolimus, cyclosporine, sirolimus, and vincristine. See, e.g., www.fda.gov/drugs/drug-interactions-labeling/drug-development-and-drug-interactions-table-substrates-inhibitors-and-inducers, accessed May 2021. In some embodiments, the patient is not administered a P-gp substrate in combination with sotorasib, wherein the P-gp substrate is a P-gp substrate with a narrow therapeutic index. Exemplary P-gp substrates with a narrow therapeutic index include, but are not limited to, digoxin, everolimus, cyclosporine, sirolimus, and vincristine.

In various embodiments, the patient has a cancer that was determined to have one or more cells expressing the $KRAS^{G12C}$ mutant protein prior to administration of sotorasib as disclosed herein. Determination of $KRAS^{G12C}$ mutant protein can be assessed as described elsewhere in this disclosure.

In some embodiments, the patient administered sotorasib in the methods described herein have been previously treated with a different anti-cancer therapy, e.g., at least one - such as one, or two, or three - other systemic cancer therapy. In some embodiments, the patient had previously been treated with one other systemic cancer therapy, such that the sotorasib therapy is a second line therapy. In some embodiments, the patient had previously been treated with two other systemic cancer therapy, such that the sotorasib therapy is a third line therapy.

In some embodiments, the prior systemic cancer therapy is not a therapy with a $KRAS^{G12C}$ inhibitor. In some embodiments, $KRAS^{G12C}$ inhibitor is sotorasib, adagrasib, GDC-6036, D-1553, JDQ443, LY3484356, BI1823911, JAB-21822, RMC-6291, or APG-1842. In certain embodiments the $KRAS^{G12C}$ inhibitor is sotorasib. In certain embodiments, the $KRAS^{G12C}$ inhibitor is adagrasib. Prior systemic cancer therapies include, but are not limited to, chemotherapies and immunotherapies. Specific contemplated prior systemic cancer therapies include anti-PD1 therapy, anti-PDL1 therapy, and platinum based chemotherapy. Some examples of anti-PD1 therapy and anti-PDL1 therapies include, but are not limited to, pembrolizumab, nivolumab, cemiplimab, tisielizumab, toripalimab, aspartalizumab, dostarlimab, retifanlimab, simtilimab, pidilizumab atezolizumab, avelumab, and durvalumab. In some embodiments the anti-PD1 therapy is cemiplimab, dostarlimab, pembrolizumab, or nivolumab. In some embodiments the anti-PDL1 therapy is adebrelimab, atezolizumab, avelumab, cosibelimab, durvalumab, envafolimab, erfonrilimab, garivulimab, lodapolimab, opucolimab, sugemalimab, socazolimab, or tagitanlimab. In some embodiments the anti-PDL1 therapy is atezolizumab, avelumab, or durvalumab. Some examples of platinum based chemotherapies include, but are not limited to, carboplatin, oxaliplatin, cisplatin, nedaplatin, satraplatin, lobaplatin, triplatin, tetranitrate, picoplatin, ProLindac, and aroplatin.

In some embodiments, the patient has previously been administered a systemic cancer therapy that is a targeted

therapy if the cancer was identified to have an actionable oncogenic driver mutation in the epidermal growth factor receptor gene (*EGFR*), anaplastic lymphoma kinase gene (*ALK*), and/or ROS proto-oncogene 1 (ROS1). Targeted therapies for *EGFR* mutations include, but are not limited to, cetuximab, panitumumab, erlotinib, gefitinib, and afatinib. Targeted therapies for *ALK* mutations include, but are not limited to, crizotinib, entrectinib, lorlatinib, repotrectinib, brigatinib, alkotinib, alectinib, ensartinib, and ceritinib. Targeted therapies for ROS1 mutations include, but are not limited to, crizotinib, entrecetinib, ensartinib, alkotinib, brigatinib, taletrectinib, cabozantinib, repotrectinib, lorlatinib, and ceritinib.

In various embodiments, the patient has an intracranial lesion that is greater than 5 mm. In some embodiments, the patient has an intracranial lesion that is greater than 10 mm.

In various embodiments, the patient exhibits an Eastern Cooperative Oncology Group (ECOG) performance status of 0 or 1 (see, e.g., Zubrod et al., 1960). Status 0 indicates fully active and able to carry on all pre-disease performance without restriction. Status 1 indicates restricted in physically strenuous activity but ambulatory and able to carry out work of a light or sedentary nature. Status 2 indicates ambulatory and capable of all selfcare but unable to carry out any work activities; up and about more than 50% of waking hours. Status 3 indicates capable of only limited selfcare, confined to bed or chair more than 50% of waking hours. Status 4 indicates completely disabled, cannot carry on any selfcare and totally confined to bed or chair. Status 5 indicates death.

Adverse Events

In some embodiments, the methods comprise administering a reduced total daily dose of sotorasib when the patient experiences an adverse event to the initial total daily dose, wherein the cancer is a *KRAS* G12C mutated cancer. For example, in some embodiments, the initial daily dose is 960 mg sotorasib and the reduced total daily dose is 480 mg sotorasib. In some embodiments, the initial daily dose is 480 mg sotorasib and the reduced total daily dose is 240 mg sotorasib. In some embodiments, the methods further comprise administering a second reduced total daily dose of sotorasib when the patient experiences an adverse event to the reduced total daily dose.

The term "adverse event or (AE)" as used herein refers to any unfavorable and unintended sign (including an abnormal laboratory finding), symptom, or disease temporally associated with the use of a medical treatment or procedure that may be considered related to the medical treatment or procedure.

In some embodiments, the adverse event is hepatotoxicity (e.g., elevation of liver enzymes), interstitial lung disease (ILD)/pneumonitis, diarrhea, and/or nausea/vomiting.

*Hepatotoxicity*

In some embodiments, the adverse event is hepatotoxicity. The term "hepatotoxicity" as used herein refers to a patient having abnormal laboratory values of liver biomarkers (e.g., alkaline phosphatase (ALP), aspartate amino transferase (AST), alanine aminotransferase (ALT), and/or total bilirubin (TBL)), when the patient had baseline levels of the liver biomarker(s) prior to sotorasib administration that were not abnormal laboratory values or were lower than those measured after administration of sotorasib.

Alanine transaminase (ALT), also called serum glutamic pyruvate transaminase (SGPT) or alanine aminotransferase (ALAT), catalyzes the transfer of an amino group from alanine to α-ketoglutarate to produce pyruvate and glutamate. When the liver is damaged, levels of ALT in the blood can rise due to the leaking of ALT into the blood from damaged or necrosed hepatocytes.

Aspartate transaminase (AST) also called serum glutamic oxaloacetic transaminase (SGOT or GOT) or aspartate aminotransferase (ASAT), catalyzes the transfer of an amino group from aspartate to α-ketoglutarate to produce oxaloacetate and glutamate. AST can increase in response to liver damage. Elevated AST also can result from damage to other sources, including red blood cells, cardiac muscle, skeletal muscle, kidney tissue, and brain tissue. The ratio of AST to ALT can be used as a biomarker of liver damage.

Bilirubin is a catabolite of heme that is cleared from the body by the liver. Conjugation of bilirubin to glucuronic acid by hepatocytes produces direct bilirubin, a water-soluble product that is readily cleared from the body. Indirect bilirubin is unconjugated, and the sum of direct and indirect bilirubin constitutes total bilirubin. Elevated total bilirubin can be indicative of liver impairment.

Alkaline phosphatase (ALP) hydrolyzes phosphate groups from various molecules and is present in the cells lining the biliary ducts of the liver. ALP levels in plasma can rise in response to liver damage and are higher in growing children and elderly patients with Paget's disease. However, elevated ALP levels usually reflect biliary tree disease.

In some embodiments, the patient is not suffering from a disorder that results in elevated liver biomarkers. Disorders associated with elevated liver biomarkers (such as AST/ALT and/or TBL values) include, but are not limited to, hepatobiliary tract disease; viral hepatitis (e.g., hepatitis A/B/C/D/E, Epstein-Barr Virus, cytomegalovirus, herpes simplex virus, varicella, toxoplasmosis, and parvovirus); right sided heart failure, hypotension or any cause of hypoxia to the liver causing ischemia; exposure to hepatotoxic agents/drugs or hepatotoxins, including herbal and dietary supplements,

plants and mushrooms; heritable disorders causing impaired glucuronidation (e.g., Gilbert's syndrome, Crigler-Najjar syndrome) and drugs that inhibit bilirubin glucuronidation (e.g., indinavir, atazanavir); alpha-one antitrypsin deficiency; alcoholic hepatitis; autoimmune hepatitis; Wilson's disease and hemochromatosis; nonalcoholic fatty liver disease including steatohepatitis; and/or non-hepatic causes (e.g., rhabdomyolysis, hemolysis).

Prior to receiving sotorasib, the baseline liver function of the patient can be assessed by various means known in the art, such as blood chemistry tests measuring biomarkers of liver function. In some embodiments, the methods described herein comprise monitoring liver biomarkers in the patient and withholding sotorasib administration in patients having > Grade 2 abnormal liver function, as assessed by levels of AST and/or ALT. In such embodiments, sotorasib administration is paused until the AST and/or ALT levels in the patient improve(s) to Grade 1 or better (baseline).

Adverse effect Grades for abnormal liver function are defined herein by the modified Common Toxicity Criteria (CTC) provided in Table 1. See the National Cancer Institute Common Terminology Criteria for Adverse Events v5.0 (NCI CTCAE) published Nov. 27, 2017 by the National Cancer Institute.

Table 1.

| Common Toxicity Criteria | | | | | |
|---|---|---|---|---|---|
| TOXICITY GRADES | | | | | |
| **TOXICITY** | **0** | **1** | **2** | **3** | **4** |
| ALT | WNL | > ULN - 3.0 x ULN, if baseline was normal; 1.5 - 3.0 x baseline is baseline was abnormal | >3-5 x ULN, if baseline was normal, >3.0 - 5.0 x baseline if baseline was abnormal | >5-20 x ULN, if baseline was normal; >5.0 - 20.0 x baseline if baseline was abnormal | >20 x ULN if baseline was normal; > 20 x baseline if baseline was abnormal |
| AST | WNL | > ULN - 3.0 x ULN if baseline was normal; 1.5 - 3.0 x baseline is baseline was abnormal | >3-5 x ULN if baseline was normal, >3.0 - 5.0 x baseline id baseline was abnormal | >5-20 x ULN if baseline was normal; >5.0 - 20.0 x baseline if baseline was abnormal | >20 x ULN if baseline was normal; > 20 x baseline if baseline was abnormal |
| Bilirubin | WNL | > ULN - 1.5 x ULN if baseline was normal; > 1.0 - 1.5 x baseline if baseline was abnormal | >1.5-3 x ULN if baseline was normal; > 1.5 - 3.0 x baseline if baseline was abnormal | >3-10 x ULN if baseline was normal; > 3.0 - 10 x baseline if baseline was abnormal | >10 x ULN if baseline was normal; > 10.0 x baseline if baseline was abnormal |
| ALP | WNL | > ULN - 2.5 x ULN if baseline was normal; 2.0- 2.5 x baseline is baseline was abnormal | >2.5 - 5.0 x ULN if baseline was normal, >2.5 - 5.0 x baseline if baseline was abnormal | >5-20 x ULN if baseline was normal; >5.0 - 20.0 x baseline if baseline was abnormal | >20 x ULN if baseline was normal; > 20 x baseline if baseline was abnormal |
| ALP = alkaline phosphatase; ALT = alanine aminotransferase; AST = aspartate aminotransferase; ULN = upper limit of normal; WNL= within normal limits | | | | | |

Grade 0 levels are characterized by biomarker levels within normal limits (WNL). "Normal" liver function, as used herein, refers to Grade 0 adverse effects. "Abnormal" liver function, as used herein, refers to Grade 1 and above adverse effects.

"Grade 1 liver function abnormalities" include elevations in ALT, AST, or ALP greater than the ULN and less than or equal to 3-times the ULN if baseline was normal; 1.5 - 3.0 x baseline is baseline was abnormal. Grade 1 liver function abnormalities also include elevations of bilirubin levels greater than the ULN and less than or equal to 1.5-times the ULN if baseline was normal; > 1.0 - 1.5 x baseline if baseline was abnormal. Grade 1 liver function abnormalities also include elevations of ALP greater than the ULN and less than or equal to 2.5-times the ULN if baseline was normal; > 2.0 - 2.5 x baseline if baseline was abnormal.

"Grade 2 liver function abnormalities" include elevations in alanine transaminase (ALT), aspartate transaminase (AST), or alkaline phosphatase (ALP) greater than 3-times and less than or equal to 5-times the upper limit of normal (ULN) if baseline was normal, >3.0 - 5.0 x baseline if baseline was abnormal. Grade 2 liver function abnormalities also include elevations of bilirubin levels greater than 1.5-times and less than or equal to 3-times the ULN if baseline was normal; > 1.5 - 3.0 x baseline if baseline was abnormal. Grade 2 liver function abnormalities also include elevations of ALP greater than

2.5-times and less than or equal to 5-times the ULN if baseline was normal; > 2.5 - 5.0 x baseline if baseline was abnormal.

"Grade 3 liver function abnormalities" include elevations in ALT, AST, or ALP greater than 5-times and less than or equal to 20-times the ULN if baseline was normal; >5.0 - 20.0 x baseline if baseline was abnormal. Grade 3 liver function abnormalities also include elevations of bilirubin levels greater than 3-times and less than or equal to 10-times the ULN if baseline was normal; > 3.0 - 10 x baseline if baseline was abnormal.

"Grade 4 liver function abnormalities" include elevations in ALT, AST, or ALP greater than 20-times the ULN if baseline was normal; > 20 x baseline if baseline was abnormal. Grade 4 liver function abnormalities also include elevations of bilirubin levels greater than 10 times the ULN if baseline was normal; > 10.0 x baseline if baseline was abnormal.

The ULN for various indicators of liver function depends on the assay used, the patient population, and each laboratory's normal range of values for the specified biomarker, but can readily be determined by the skilled practitioner. Exemplary values for normal ranges for a healthy adult population are set forth in Table 2 below. See Cecil Textbook of Medicine, pp. 2317-2341, W.B. Saunders & Co. (1985).

Table 2. - Upper Limit of Normal (ULN) Values

| | |
|---|---|
| ALT | 8-20 U/L |
| AST | 8-20 U/L |
| Bilirubin | 0.2-1 mg/dL<br>3.4-17.1 μmol/L |
| ALP | 20-70 U/L |

In any of the methods described herein, the total daily dose of sotorasib is reduced (e.g., from 960 mg to 480 mg, or from 480 mg to 240 mg) when the AST and/or ALT level(s) in the patient is/are elevated, e.g. to a Grade 2 or Grade 3 level, where the baseline AST and/or ALT levels of the patient were below Grade 2 or Grade 3 levels. In some embodiments, the total daily dose of sotorasib is reduced (e.g., from 960 mg to 480 mg, or from 480 mg to 240 mg), when the AST and/or ALT level(s) in the patient is/are elevated is to a Grade 1 level, wherein the baseline AST and/or ALT levels of the patient were below Grade 1 levels.

Alternatively, in any of the methods disclosed herein, the total daily dose of sotorasib is reduced (e.g., from 960 mg to 480 mg, or from 480 mg to 240 mg) when (1) AST and bilirubin levels in the patient are elevated, or (2) when AST or ALP levels in the patient are elevated, or (3) when ALT and bilirubin levels in the patient are elevated, or (4) when ALT and ALP levels in the patient are elevated, or (5) when bilirubin and ALP levels in the patient are elevated, e.g., to a Grade 1, Grade 2, Grade 3 or Grade 4 level, wherein the baseline AST, bilirubin, ALP, and/or ALT levels of the patient were below Grade 1, Grade 2, Grade 3 or Grade 4 levels, respectively. Alternatively, in any of the methods disclosed herein, three biomarkers of liver function may be elevated in the patient (e.g., ALT and AST and bilirubin, or ALT and AST and ALP) to a Grade 1, Grade 2, Grade 3 or Grade 4 level, wherein the baseline biomarker levels of the patient were below Grade 1, Grade 2, Grade 3 or Grade 4 levels, respectively.

In some embodiments, the total daily dose of sotorasib is reduced (e.g., from 960 mg to 480 mg, or from 480 mg to 240 mg) when the level of ALT and/or AST is greater than about 3 times compared to the upper limit of normal (ULN). In a related embodiment, the abnormal level of ALT and/or AST is greater than about 3- to about 5-fold increase compared to the upper limit of normal (ULN), i.e. a "Grade 2 abnormality". In some embodiments, where the patient has an abnormal baseline, the Grade 2 abnormality is an abnormal level of ALT and/or AST greater than about 3-fold to about 5-fold increase compared to baseline. In some embodiments, the abnormal level of ALP is greater than about 2.5- to about 5-fold increase compared to the upper limit of normal (ULN), i.e., a "Grade 2 abnormality". In some embodiments, where the patient has an abnormal baseline, the Grade 2 abnormality is an abnormal level of ALP greater than about 2.5-fold to about 5-fold increase compared to baseline. In some embodiments, the abnormal level of bilirubin is greater than about 1.5- to about 3-fold increase compared to the upper limit of normal (ULN), i.e., a "Grade 2 abnormality". In some embodiments, where the patient has an abnormal baseline, the Grade 2 abnormality is an abnormal level of bilirubin greater than about 1.5-fold to about 3-fold increase compared to baseline.

In some embodiments, the total daily dose of sotorasib is reduced (e.g., from 960 mg to 480 mg, or from 480 mg to 240 mg) when the level of ALT and/or AST is greater than about 5 times compared to the upper limit of normal (ULN). In some embodiments, the total daily dose is reduced when the level of ALT, AST, or ALP is greater than about 5- to about 20-fold increase compared to the upper limit of normal (ULN), i.e. a "Grade 3 abnormality". In some embodiments, where the patient has an abnormal baseline, the Grade 3 abnormality is an abnormal level of ALT and/or AST greater than about 5-fold to about 20-fold increase compared to baseline. In some embodiments, the abnormal level of ALP is greater than about 5- to about 20-fold increase compared to the upper limit of normal (ULN), i.e., a "Grade 3 abnormality". In some embodiments, where the patient has an abnormal baseline, the Grade 3 abnormality is an abnormal level of ALP greater than about 5-fold to about 20-fold increase compared to baseline. In some embodiments, the total daily dose is reduced

when the level of bilirubin is greater than about 3- to about 10-fold increase compared to the upper limit of normal (ULN), i.e., a "Grade 3 abnormality". In some embodiments, where the patient has an abnormal baseline, the Grade 3 abnormality is an abnormal level of bilirubin greater than about 3-fold to about 10-fold increase compared to baseline.

In some embodiments, the total daily dose of sotorasib is reduced (e.g., from 960 mg to 480 mg, or from 480 mg to 240 mg) when the level of ALT and/or AST is greater than about 20 times compared to the upper limit of normal (ULN) (i.e., a "Grade 4 abnormality"). In some embodiments, where the patient has an abnormal baseline, the Grade 4 abnormality is an abnormal level of ALT and/or AST greater than about 20-fold increase compared to baseline. In some embodiments, the abnormal level of ALP is greater than about 20-fold increase compared to the upper limit of normal (ULN), i.e., a "Grade 4 abnormality". In some embodiments, where the patient has an abnormal baseline, the Grade 4 abnormality is an abnormal level of ALP greater than about 20-fold increase compared to baseline. In some embodiments, the total daily dose is reduced when the level of bilirubin is greater than about 10-fold increase compared to the upper limit of normal (ULN), i.e., a "Grade 4 abnormality". In some embodiments, where the patient has an abnormal baseline, the Grade 4 abnormality is an abnormal level of bilirubin greater than about 10-fold increase compared to baseline.

In some embodiments, the methods described herein further comprise increasing the total dose of sotorasib (e.g., from 240 mg to 480mg, or from 480 mg to 960 mg) when liver biomarker(s) in the patient has improved to a Grade 1 or better (e.g., baseline).

*Nausea/Vomiting*

In some embodiments, the adverse event is nausea or vomiting. In some embodiments, the nausea/vomiting is present despite appropriate supportive care (e.g., anti-emetic therapy). "Nausea" as used herein refers to a disorder characterized by a queasy sensation and/or the urge to vomit.

Adverse effect Grades for nausea and vomiting are defined herein by the modified Common Toxicity Criteria (CTC) provided in Table 3. See the National Cancer Institute Common Terminology Criteria for Adverse Events v5.0 (NCI CTCAE) published Nov. 27, 2017 by the National Cancer Institute.

Table 3.

| | Grade 1 | Grade 2 | Grade 3 | Grade 4 |
|---|---|---|---|---|
| Nausea | Loss of appetite without alteration in eating habits | Oral intake decreased without significant weight loss, dehydration or malnutrition | Inadequate oral caloric or fluid intake; tube feeding, total parenteral nutrition (TPN), or hospitalization indicated | -- |
| Vomiting | Intervention not indicated | Outpatient IV hydration; medical intervention indicated | Tube feeding, TPN, or hospitalization indicated | Life-threatening consequences |

In some embodiments, the methods described herein comprise withholding sotorasib administration in a patient having > Grade 3 nausea until the patient has improved to ≤ Grade 1 or baseline. In some embodiments, once the patient has improved to ≤ Grade 1 or baseline, the methods comprise administering a reduced total daily dose of sotorasib (e.g., from 960 mg to 480 mg, or from 480 mg to 240 mg) to the patient.

In some embodiments, the methods described herein comprise withholding sotorasib administration in a patient having > Grade 3 vomiting until the vomiting improves to ≤ Grade 1 or baseline. In some embodiments, once the patient has improved to ≤ Grade 1 or baseline, the methods comprise administering a reduced total daily dose of sotorasib (e.g., from 960 mg to 480 mg, or from 480 mg to 240 mg) to the patient.

In some embodiments, the methods described herein further comprise increasing the total dose of sotorasib (e.g., from 240 mg to 480mg, or from 480 mg to 960 mg) when nausea in the patient has improved to a Grade 1 or better (e.g., baseline).

*Diarrhea*

In some embodiments, the adverse event is diarrhea. In some embodiments, the diarrhea is present despite appropriate supportive care (e.g., anti-diarrheal therapy).

Adverse effect Grades for diarrhea are defined herein by the modified Common Toxicity Criteria (CTC) provided in Table 4. See the National Cancer Institute Common Terminology Criteria for Adverse Events v5.0 (NCI CTCAE) published Nov. 27, 2017 by the National Cancer Institute

Table 4.

| | Grade 1 | Grade 2 | Grade 3 | Grade 4 |
|---|---|---|---|---|
| Diarrhea | Increase of <4 stools per day over baseline; mild increase in ostomy output compared to baseline | Increase of 4-6 stools per day over baseline; moderate increase in ostomy output compared to baseline; limiting instrumental activities of daily living (ADL) | Increase of ≥ 7 stools per day over baseline; hospitalization indicated; severe increase in ostomy output compared to baseline; limiting self care ADL | Life-threatening consequences; urgent intervention indicated |

In some embodiments, the methods described herein comprise withholding sotorasib administration in a patient having > Grade 3 diarrhea until the patient has improved to ≤ Grade 1 or baseline. In some embodiments, once the patient has improved to ≤ Grade 1 or baseline, the methods comprise administering a reduced total daily dose of sotorasib (e.g., from 960 mg to 480 mg, or from 480 mg to 240 mg) to the patient.

In some embodiments, the methods described herein further comprise increasing the total dose of sotorasib (e.g., from 240 mg to 480mg, or from 480 mg to 960 mg) when diarrhea in the patient has improved to a Grade 1 or better (e.g., baseline).

Interstitial Lung Disease

In some embodiments, the adverse event is interstitial lung disease (ILD) or pneumonitis. In cases where ILD or pneumonitis is suspected at any grade level, sotorasib is withheld. In cases where ILD or pneumonitis is confirmed, and no other causes of the ILD or pneumonitis is identified, sotorasib is permanently discontinued.

**Response to Sotorasib Therapy**

Response rates or results for patients administered sotorasib in the methods disclosed herein can be measured in a number of ways, after the patient has been taking sotorasib for a suitable length of time. In various embodiments, a patient is administered sotorasib (960 mg, 480 mg, or 240 mg once daily) for at least 1 month, at least 2 months, at least 3 months, at least 4 months, at least 5 months, at least 6 months, at least 7 months, at least 8 months, at least 9 months, at least 10 months, at least 11 months, at least 12 months, at least 15 months, at least 18 months, at least 21 months, or at least 23 months, e.g., for 1 month, 2 months, 3 months, 4 months, 5 months, 6 months, 7 months, 8 months, 9 months, 10 months, 11 months, 12 months, 15 months, 18 months, 21 months, or 24 months. In various embodiments, the patient is administered sotorasib (960 mg, 480 mg, or 240 mg once daily) for at least 1 month. In various embodiments, the patient is administered sotorasib (960 mg, 480 mg, or 240 mg once daily) for at least 3 months. In various embodiments, the patient is administered sotorasib (960 mg, 480 mg, or 240 mg once daily) for at least 6 months.

In various embodiments, a patient is administered sotorasib (480 mg or 240 mg twice daily) for at least 1 month, at least 2 months, at least 3 months, at least 4 months, at least 5 months, at least 6 months, at least 7 months, at least 8 months, at least 9 months, at least 10 months, at least 11 months, at least 12 months, at least 15 months, at least 18 months, at least 21 months, or at least 23 months, e.g., for 1 month, 2 months, 3 months, 4 months, 5 months, 6 months, 7 months, 8 months, 9 months, 10 months, 11 months, 12 months, 15 months, 18 months, 21 months, or 24 months. In various embodiments, the patient is administered sotorasib (480 mg or 240 mg twice daily) for at least 1 month. In various embodiments, the patient is administered sotorasib (480 mg or 240 mg twice daily) for at least 3 months. In various embodiments, the patient is administered sotorasib (480 mg or 240 mg twice daily) for at least 6 months.

The patient can respond to the sotorasib therapy as measured by at least a stable disease (SD), as determined by RECIST 1.1 protocol (Eisenhauer, et al., 2009). An at least stable disease is one that is a stable disease, has shown a partial response (PR) or has shown a complete response (CR) (i.e., "at least SD" = SD+PR+CR, often referred to as disease control). In various embodiments, the stable disease is neither sufficient shrinkage to qualify for partial response (PR) nor sufficient increase to qualify for progressive disease (PD). In various embodiments, the patient exhibits at least a partial response (i.e., "at least PR" = PR+CR, often referred to as objective response).

Response can be measured by one or more of decrease in tumor size, suppression or decrease of tumor growth, decrease in target or tumor lesions, delayed time to progression, no new tumor or lesion, a decrease in new tumor formation, an increase in survival or progression-free survival (PFS), and no metastases. In various embodiments, the progression of a patient's disease can be assessed by measuring tumor size, tumor lesions, or formation of new tumors or lesions, by assessing the patient using a computerized tomography (CT) scan, a positron emission tomography (PET) scan, a magnetic resonance imaging (MRI) scan, an X-ray, ultrasound, or some combination thereof.

Progression free survival (PFS) can be assessed as described in the RECIST 1.1 protocol. In various embodiments, the patient exhibits a PFS of at least 1 month. In various embodiments, the patient exhibits a PFS of at least 3 months. In some embodiments, the patient exhibits a PFS of at least 6 months.

Response can also be measured by the size of intracranial lesion as assessed by Response Assessment in Neuro-Oncology Brain Metastases (RANO-BM) (Lin et al, 2015). RANO-BM is an extension of the Response Evaluation Criteria in Solid Tumors (RECIST) 1.1 (Eisenhauer et al, 2009) and the Response Assessment in Neuro oncology (RANO) (Wen et al, 2010) response assessment criteria for high-grade gliomas.

Intracranial progression free survival (PFS) can be assessed as described in the RANO-BM protocol. In various embodiments, the patient exhibits a intracranial PFS of at least 1 month. In various embodiments, the patient exhibits an intracranial PFS of at least 3 months. In some embodiments, the patient exhibits an intracranial PFS of at least 6 months.

Additional means for assessing response are described in detail in the examples below, and can generally be applied to the methods disclosed herein.

***KRAS*** *G12C* Cancers

The methods described herein comprise treating a cancer comprising a KRAS G12C mutation in a patient with active brain metastases, wherein the methods comprise administering sotorasib to the patient in an amount effective to treat the cancer. Without wishing to be bound by any particular theory, the following is noted: sotorasib is a small molecule that specifically and irreversibly inhibits $KRAS^{G12C}$ (Hong et al., 2020). Hong et al. report that "[p]reclinical studies showed that [sotorasib] inhibited nearly all detectable phosphorylation of extracellular signal-regulated kinase (ERK), a key downstream effector of KRAS, leading to durable complete tumor regression in mice bearing KRAS p.G12C tumors." (id., see also Canon et al., 2019, and Lanman et al., 2020). Thus, in various embodiments, sotorasib at a total daily dose of 240 mg is disclosed for use in treating cancer, wherein one or more cells express KRAS G12C mutant protein.

Sotorasib was evaluated in a Phase 1 dose escalation and expansion trial with 129 subjects having histologically confirmed, locally advanced or metastatic cancer with the KRAS p.G12C mutation identified by local molecular testing on tumor tissues, including 59 subjects with non-small cell lung cancer, 42 subjects with colorectal cancer, and 28 subjects with other tumor types (Hong et al., 2020, at page 1208-1209). Hong et al. report a disease control rate (95% CI) of 88.1% for non-small cell lung cancer, 73.8% for colorectal cancer and 75.0% for other tumor types (Hong et al., 2020, at page 1213, Table 3). The cancer types showing either stable disease (SD) or partial response (PR) as reported by Hong et al. were non-small cell lung cancer, colorectal cancer, pancreatic cancer, appendiceal cancer, endometrial cancer, cancer of unknown primary, ampullary cancer, gastric cancer, small bowel cancer, sinonasal cancer, bile duct cancer, or melanoma (Hong et al., 2020, at page 1212 (Figure A), and Supplementary Appendix (page 59 (Figure S5) and page 63 (Figure S6)).

*KRAS G12C* mutations occur with the alteration frequencies shown in the table below (Cerami et al., 2012; Gao et al., 2013). For example, the table shows that 11.6% of subjects with non-small cell lung cancer have a cancer, wherein one or more cells express KRAS G12C mutant protein. Accordingly, sotorasib, which specifically and irreversibly bind to $KRAS^{G12C}$ is useful for treatment of subjects having a cancer, including, but not limited to the cancers listed in Table 5 below.

Table 5

| Cancer Type | Alteration Frequency |
|---|---|
| Non-Small Cell Lung Cancer | 11.6 |
| Small Bowel Cancer | 4.2 |
| Appendiceal Cancer | 3.6 |
| Colorectal Cancer | 3.0 |
| Cancer of Unknown Primary | 2.9 |
| Endometrial Cancer | 1.3 |
| Mixed Cancer Types | 1.2 |
| Pancreatic Cancer | 1.0 |
| Hepatobiliary Cancer | 0.7 |
| Small Cell Lung Cancer | 0.7 |
| Cervical Cancer | 0.7 |
| Germ Cell Tumor | 0.6 |

(continued)

| Cancer Type | Alteration Frequency |
|---|---|
| Ovarian Cancer | 0.5 |
| Gastrointestinal Neuroendocrine Tumor | 0.4 |
| Bladder Cancer | 0.4 |
| Myelodysplastic/Myeloproliferative Neoplasms | 0.3 |
| Head and Neck Cancer | 0.3 |
| Esophagogastric Cancer | 0.2 |
| Soft Tissue Sarcoma | 0.2 |
| Mesothelioma | 0.2 |
| Thyroid Cancer | 0.1 |
| Leukemia | 0.1 |
| Melanoma | 0.1 |

In various embodiments, the cancer is a solid tumor. In various embodiments, the cancer is non-small cell lung cancer, small bowel cancer, appendiceal cancer, colorectal cancer, cancer of unknown primary, endometrial cancer, mixed cancer types, pancreatic cancer, hepatobiliary cancer, small cell lung cancer, cervical cancer, germ cell cancer, ovarian cancer, gastrointestinal neuroendocrine cancer, bladder cancer, myelodysplastic/myeloproliferative neoplasms, head and neck cancer, esophagogastric cancer, soft tissue sarcoma, mesothelioma, thyroid cancer, leukemia, or melanoma. In some embodiments, the cancer is small bowel cancer, appendiceal cancer, endometrial cancer, hepatobiliary cancer, small cell lung cancer, cervical cancer, germ cell tumor, ovarian cancer, gastrointestinal neuroendocrine tumor, bladder cancer, myelodysplastic/myeloproliferative neoplasms, head and neck cancer, esophagogastric cancer, soft tissue sarcoma, mesothelioma, thyroid cancer, leukemia, or melanoma. In various embodiments, the cancer is non-small cell lung cancer, and in some specific embodiments, metastatic or locally advanced and unresectable non-small cell lung cancer. In various embodiments, the cancer is colorectal cancer. In some embodiments, the cancer is pancreatic cancer.

**Methods of Detecting KRAS, STK11, KEAP1 EGFR, ALK, and/or ROS1 Mutation Status**

The presence or absence of *G12C, STK11, KEAP1, EGFR, ALK* and/or *ROS1* mutations in a cancer as described herein can be determined using methods known in the art. Determining whether a tumor or cancer comprises a mutation can be undertaken, for example, by assessing the nucleotide sequence encoding the protein, by assessing the amino acid sequence of the protein, or by assessing the characteristics of a putative mutant protein or any other suitable method known in the art. The nucleotide and amino acid sequences sequence of wild-type human KRAS (nucleotide sequence set forth in Genbank Accession No. BC010502; amino acid sequence set forth in Genbank Accession No. AGC09594 ), STK11 (Gene ID: 6794; available at https://www.ncbi.nlm.nih.gov/gene/6794; accessed January 2020), KEAP1 (Gene ID: 9817; available at www.ncbi.nlm.nih.gov/gene/9817; accessed January 2020), EGFR (Gene ID: 1956; available at www.ncbi.nlm.nih.gov/gene/1956; accessed March 2021), ALK (Gene ID: 238; available at https://www.ncbi.nlm.nih.gov/gene/238; accessed March 2021), and ROS1 (Gene ID: 6098; available at https://www.ncbi.nlm.nih.gov/gene/6098; accessed March 2021) are known in the art.

Methods for detecting a mutation include, but are not limited to, polymerase chain reaction-restriction fragment length polymorphism (PCR-RFLP) assays, polymerase chain reaction-single strand conformation polymorphism (PCR-SSCP) assays, real-time PCR assays, PCR sequencing, mutant allele-specific PCR amplification (MASA) assays, direct and/or next generation-based sequencing, primer extension reactions, electrophoresis, oligonucleotide ligation assays, hybridization assays, TaqMan assays, SNP genotyping assays, high resolution melting assays and microarray analyses. In some embodiments, samples are evaluated for mutations, such as the *KRAS G12C* mutation, by real-time PCR. In real-time PCR, fluorescent probes specific for a certain mutation, such as the *KRAS G12C* mutation, are used. When a mutation is present, the probe binds and fluorescence is detected. In some embodiments, the mutation is identified using a direct sequencing method of specific regions in the gene. This technique identifies all possible mutations in the region sequenced. In some embodiments, gel electrophoresis, capillary electrophoresis, size exclusion chromatography, sequencing, and/or arrays can be used to detect the presence or absence of insertion mutations. In some embodiments, the methods include, but are not limited to, detection of a mutant using a binding agent (e.g., an antibody) specific for the

mutant protein, protein electrophoresis and Western blotting, and direct peptide sequencing.

In some embodiments, multiplex PCR-based sequencing is used for mutation detection, and can include a number of amplicons that provides improved sensitivity of detection of one or more genetic biomarkers. For example, multiplex PCR-based sequencing can include about 60 amplicons (e.g., 50, 51, 52, 53, 54, 55, 56, 57, 58, 59, 60, 61, 62, 63, 64, 65, 66, 67, 68, 69, or 70 amplicons). In some embodiments, multiplex PCR-based sequencing can include 61 amplicons. Amplicons produced using multiplex PCR-based sequencing can include nucleic acids having a length from about 15 bp to about 1000 bp (e.g., from about 25 bp to about 1000 bp, from about 35 bp to about 1000 bp, from about 50 bp to about 1000 bp, from about 100 bp to about 1000 bp, from about 250 bp to about 1000 bp, from about 500 bp to about 1000 bp, from about 750 bp to about 1000 bp, from about 15 bp to about 750 bp, from about 15 bp to about 500 bp, from about 15 bp to about 300 bp, from about 15 bp to about 200 bp, from about 15 bp to about 100 bp, from about 15 bp to about 80 bp, from about 15 bp to about 75 bp, from about 15 bp to about 50 bp, from about 15 bp to about 40 bp, from about 15 bp to about 30 bp, from about 15 bp to about 20 bp, from about 20 bp to about 100 bp, from about 25 bp to about 50 bp, or from about 30 bp to about 40 bp). For example, amplicons produced using multiplex PCR-based sequencing can include nucleic acids having a length of about 33 bp.

In some embodiments, the presence of one or more mutations present in a sample obtained from a patient is detected using sequencing technology (e.g., a next-generation sequencing technology). A variety of sequencing technologies are known in the art. For example, methods for detection and characterization of circulating tumor DNA in cell-free DNA can be described elsewhere (see, e.g., Haber and Velculescu, 2014). Non-limiting examples of such techniques include SafeSeqs (see, e.g., Kinde et al., 2011), OnTarget (see, e.g., Forshew et al., 2012), and TamSeq (see, e.g., Thompson et al., 2012).

In some embodiments, the presence of one or more mutations present in a sample obtained from a patient is detected using droplet digital PCR (ddPCR), a method that is known to be highly sensitive for mutation detection. In some embodiments, the presence of one or more mutations present in a sample obtained from a patient is detected using other sequencing technologies, including but not limited to, chain-termination techniques, shotgun techniques, sequencing-by-synthesis methods, methods that utilize microfluidics, other capture technologies, or any of the other sequencing techniques known in the art that are useful for detection of small amounts of DNA in a sample (e.g., ctDNA in a cell-free DNA sample).

In some embodiments, the presence of one or more mutations present in a sample obtained from a patient is detected using array-based methods. For example, the step of detecting a genetic alteration (e.g., one or more genetic alterations) in cell-free DNA is performed using a DNA microarray. In some embodiments, a DNA microarray can detect one more of a plurality of cancer cell mutations. In some embodiments, cell-free DNA is amplified prior to detecting the genetic alteration. Non-limiting examples of array-based methods that can be used in any of the methods described herein, include: a complementary DNA (cDNA) microarray (see, e.g., Kumar et al. 2012; Laere et al. 2009; Mackay et al. 2003; Alizadeh et al. 1996), an oligonucleotide microarray (see, e.g., Kim et al. 2006; Lodes et al. 2009), a bacterial artificial chromosome (BAC) clone chip (see, e.g., Chung et al. 2004; Thomas et al. 2005), a single-nucleotide polymorphism (SNP) microarray (see, e.g., Mao et al. 2007; Jasmine et al. 2012), a microarray-based comparative genomic hybridization array (array-CGH) (see, e.g., Beers and Nederlof, 2006; Pinkel et al. 2005; Michels et al. 2007), a molecular inversion probe (MIP) assay (see, e.g., Wang et al. 2012; Lin et al. 2010). In some embodiments, the cDNA microarray is an Affymetrix microarray (see, e.g., Irizarry 2003; Dalma-Weiszhausz et al. 2006), a NimbleGen microarray (see, e.g., Wei et al. 2008; Albert et al. 2007), an Agilent microarray (see, e.g., Hughes et al. 2001), or a BeadArray array (see, e.g., Liu et al. 2017). In some embodiments, the oligonucleotide microarray is a DNA tiling array (see, e.g., Mockler and Ecker, 2005; Bertone et al. 2006). Other suitable array-based methods are known in the art.

Methods for determining whether a tumor or cancer comprises a mutation can use a variety of samples. In some embodiments, the sample is taken from a patient having a tumor or cancer. In some embodiments, the sample is a fresh tumor/cancer sample. In some embodiments, the sample is a frozen tumor/cancer sample. In some embodiments, the sample is a formalin-fixed paraffin-embedded (FFPE) sample. In some embodiments, the sample is a circulating cell-free DNA and/or circulating tumor cell (CTC) sample. In some embodiments, the sample is processed to a cell lysate. In some embodiments, the sample is processed to DNA or RNA. In a certain embodiment, the sample is acquired by resection, core needle biopsy (CNB), fine needle aspiration (FNA), collection of urine, or collection of hair follicles. In some embodiments, a liquid biopsy test using whole blood or cerebral spinal fluid may be used to assess mutation status.

In various embodiments, a test approved by a regulatory authority, such as the US Food and Drug Administration (FDA), is used to determine whether the patient has a mutation, e.g., a KRAS$^{G12C}$ mutated cancer, or whether the tumor or tissue sample obtained from such patient contains cells with a mutation. In some embodiments, the test for a *KRAS* mutation used is therascreen® KRAS RGQ PCR Kit (Qiagen). The therascreen® KRAS RGQ PCR Kit is a real-time qualitative PCR assay for the detection of 7 somatic mutations in codons 12 and 13 of the human KRAS oncogene (G12A, G12D, G12R, G12C, G12S, G12V, and G13D) using the Rotor-Gene Q MDx 5plex HRM instrument. The kit is intended for use with DNA extracted from FFPE samples of NSCLC samples acquired by resection, CNB, or FNA. Mutation testing for *STK11, KEAP1, EGFR, ALK* and/or *ROS1* can be conducted with commercially available tests, such as the Resolution

Bioscience Resolution ctDx LungTM assay that includes 24 genes (including those actionable in NSCLC). Tissue samples may be tested using Tempus xT 648 panel.

In some embodiments, the cancer has been identified as having a *KRAS G12C* mutation. In some embodiments, the cancer has been identified as having a mutation of *STK11,* e.g., a loss-of-function mutation. In some embodiments, the cancer has been identified as having a mutation of *KEAP1,* e.g., a loss-of-function mutation. In some embodiments, the cancer has been identified as having wild-type *STK11.* In some embodiments, the cancer has been identified as having wild-type *KEAP1.*

In various embodiments, the cancer has been identified as having a loss-of-function mutation of *STK11* and wild-type *KEAP1.* In some embodiments, the cancer has been identified as having a loss-of-function mutation of *STK11* and a loss-of-function mutation of *KEAP1.* In some embodiments, the cancer has been identified as having wild-type of *STK11* and wild-type *KEAP1.* In some embodiments, the cancer has been identified as having wild type of *STK11* and a loss-of-function mutation of *KEAP1.*

The term "loss-of-function mutation" as used herein refers to a mutation (e.g., a substitution, deletion, truncation, or frameshift mutation) that results in expression of a mutant protein that no longer exhibits wild-type activity (e.g., reduced or eliminated wild-type biological activity or enzymatic activity), results in expression of only a fragment of the protein that no longer exhibits wild-type activity, or results in no expression of the wild-type protein. For example, a loss-of-function mutation affecting the *STK11* gene in a cell may result in the loss of expression of the STK11 protein, expression of only a fragment of the STK11 protein, or expression of the STK11 protein that exhibits diminished or no enzymatic activity (e.g., no serine/threonine kinase enzymatic activity) in the cancerous cell. Similarly, a loss-of-function mutation affecting the *KEAP1* gene in a cell may result in the loss of expression of the KEAP1 protein, expression of only a fragment of the KEAP1 protein, or expression of a KEAP1 protein that exhibits diminished or no activity (e.g., inability to interact with or activate Nuclear factor erythroid 2-related factor 2 (NRF2)) in the cell.

**Methods of Detecting PD-L1 Protein Expression**

PD-L1 expression can be determined by methods known in the art. For example, PD-L1 expression can be detected using PD-L1 IHC 22C3 pharmDx, an FDA-approved in vitro diagnostic immunohistochemistry (IHC) test developed by Dako and Merck as a companion test for treatment with pembrolizumab. This is qualitative assay using Monoclonal Mouse Anti-PD-L1, Clone 22C3 PD-L1 and EnVision FLEX visualization system on Autostainer Lin 48 to detect PD-L1 in FFPE samples, such as human non-small cell lung cancer tissue. Expression levels can be measured using the tumor proportion score (TPS), which measures the percentage of viable tumor cells showing partial or complete membrane staining at any intensity. Staining can show PD-L1 expression from 0% to 100%.

PD-L1 expression can also be detected using PD-L1 IHC 28-8 pharmDx, the FDA- approved in vitro diagnostic immunohistochemistry (IHC) test developed by Dako and Bristol-Myers Squibb as a companion test for treatment with nivolumab. This qualitative assay uses the Monoclonal rabbit anti-PD-L1, Clone 28-8 and EnVision FLEX visualization system on Autostainer Lin 48 to detect PD-L1 in formalin-fixed, paraffin-embedded (FFPE) human cancer tissue.

Other commercially available tests for PD-L1 detection include the Ventana SP263 assay (developed by Ventana in collaboration with AstraZeneca) that utilizes monoclonal rabbit anti- PD-L1, Clone SP263 and the Ventana SP142 Assay (developed by Ventana in collaboration with Genentech/Roche) that uses rabbit monoclonal anti-PD-L1 clone SP142.

In some embodiments, a test approved by a regulatory authority, such as the US Food and Drug Administration (FDA), is used to determine the PD-L1 TPS of a cancer as disclosed herein. In various embodiment, the PD-L1 TPS is determined using a immunohistochemistry (IHC) test. In some embodiments, the IHC test is the PD-L1 IHC 22C3 pharmDx test. In various embodiments, the IHC test conducted with samples acquired by, for example, resection, CNB, or FNA.

In various embodiment, the patient has a PD-L1 TPS of less than 100%, 95%, 90%, 85%, 80%, 75%, 70%, 65%, 60%, 50%, 55%, 50%, 45%, 40%, 35%, 30%, 25%, 20%, 15%, 10%, 9%, 8%, 7%, 6%, 5%, 4%, 3%, 2%, or 1%. In various embodiments, the patient has a PD-L1 TPS of less than 50%, or less than 1%. In various embodiments, the patient has a PD-L1 TPS of more than or equal to 95%, 90%, 85%, 80%, 75%, 70%, 65%, 60%, 50%, 55%, 50%, 45%, 40%, 35%, 30%, 25%, 20%, 15%, 10%, 9%, 8%, 7%, 6%, 5%, 4%, 3%, 2%, or 1%. In various embodiments, the patient has a PD-L1 TPS of less than or equal to 100%, 95%, 90%, 85%, 80%, 75%, 70%, 65%, 60%, 50%, 55%, 50%, 45%, 40%, 35%, 30%, 25%, 20%, 15%, 10%, 9%, 8%, 7%, 6%, 5%, 4%, 3%, 2%, or 1%. In various embodiments, the patient has a PD-L1 TPS of less than or equal to 50%, or less than or equal to 1%. In various embodiments, the patient has a PD-L1 TPS of more than 95%, 90%, 85%, 80%, 75%, 70%, 65%, 60%, 50%, 55%, 50%, 45%, 40%, 35%, 30%, 25%, 20%, 15%, 10%, 9%, 8%, 7%, 6%, 5%, 4%, 3%, 2%, or 1%. In various embodiments, the patient has a PD-L1 TPS score a range bound by any of the values cited in the foregoing embodiments. For example, the patient has a PD-L1 TPS score in the range of less than 50% and more than or equal to 1%, less than or equal to 50% and more than 1%, less than or equal to 50% and more than or equal to 1%, or less than 50% and more than 1%.

In various embodiments, the patient has a PD-L1 TPS score in the range of less than 50% and more than or equal to 1%. In some embodiments, the patient has a PD-L1 TPS score in the range of more than or equal to 0% and less than 1%.

In some embodiments, the patient has a PD-L1 TPS score in the range of more than 50% and less than or equal to 100%. In some embodiments, the patient has a PD-L1 TPS score of less than 1%. In some embodiments, the patient as a PD-L1 TPS score of 1-49%. In some embodiments, the patient has a PD-L1 TPS score of 50% or greater (i.e., 50%-100%).

## EXAMPLES

### Example 1 - Sotorasib Monotherapy in Patients with NSCLC with KRAS p. G12C Mutation with Brain Metastases

Without wishing to be bound by any particular theory, the following is noted: Sotorasib at 960 mg QD was shown to be safe and effective under study conditions under Study 20170543 (CodeBreak100). Brain metastases are common in patients with non-small cell lung cancer (NSCLC), occurring in 20% to 50% of patients (Offin et al 2019; Villano et al, 2015). Nevertheless, patients with brain metastases are often excluded from clinical trials due to concerns of poor functional status, increased risk of toxicity, poorer prognosis, and shorter life expectancy. Patients with active brain metastases were not included in Study 20170543. However, treatment with sotorasib may induce an antitumor immune response through activation of T cells that recognize tumor antigens (Canon et al, 2019). The infiltration of activated T cell from the circulation to the brain may provide intracranial antitumor activity. Based on these data, sotorasib may provide an intracranial response through a mechanism similar to systemic disease control.

A multicenter, randomized, open-label study is set up to evaluate the safety and efficacy of sotorasib as monotherapy in subjects with KRAS p.G12C mutant advanced NSCLC and active brain metastases. Sotorasib is monitored as a monotherapy with either once daily (QD, Cohort A), or twice daily (BID, Cohort B) dosing. Approximately 70 subjects are enrolled and randomized to receive sotorasib either once daily (Cohort A) or twice daily (Cohort B). The number of subjects enrolled in Part 1 will be up to 30. Approximately 40 subject will be enrolled in Part 2.

Cohort A assesses the safety of sotorasib on a QD dosing schedule at up to 960 mg. Cohort B assesses the safety of sotorasib on a BID dosing schedule at up to 480 mg BID.

The dose levels for Cohort A are:

Dose level A1: Sotorasib 960 mg QD

Dose level A-1: Sotorasib 480 mg QD

Dose level A-2: Sotorasib 240 mg QD

The dose levels for Cohort B are:

Dose level B1: Sotorasib 480 mg BID

Dose level B-1: Sotorasib 240 mg BID

Dose level B-2: Sotorasib 240 mg QD

Note: Dose level B-2 is only explored if Dose level A-2 has not already started enrollment.

Cohorts A and B are enrolled concurrently. Dose exploration in Part 1 begins with 3 to 6 subjects treated at Cohort A Dose Level A1 and 3 to 6 subjects treated at Cohort B Dose Level B1. The study dose-limiting toxicity (DLT) period is 21 days. Depending on the observed safety data, the following may occur: (1) dose de-escalation within each cohort, or (2) additional enrollment to a certain dose level up to a maximum of 10 evaluable subjects.

Guideline for Dose De-escalation

| Number of evaluable[a] subjects | Number of DLT observed at current dose level | Guideline for dose de-escalation |
|---|---|---|
| 3 | ≤ 1 | Stay at current dose level |
| 3 | ≥ 2 | De-escalate or stop enrollment at current dose level[b] |

(continued)

| Number of evaluable[a] subjects | Number of DLT observed at current dose level | Guideline for dose de-escalation |
|---|---|---|
| 4 | ≤ 1 | Stay at current dose level |
| 4 | ≥ 2 | De-escalate or stop enrollment at current dose level[b] |
| 5 | ≤ 1 | Stay at current dose level |
| 5 | ≥ 2 | De-escalate or stop enrollment at current dose level[b] |
| 6 | ≤ 1 | Consider re-escalate dose level or initiate part 2[b] |
| 6 | ≥ 2 | De-escalate or stop enrollment at current dose level[c] |
| 7 or 8[c] | ≤ 1 | Consider re-escalate dose level or initiate part 2[b] |
| 7 or 8[c] | 2 | Stay at current dose level |
| 7 or 8[c] | ≥ 3 | De-escalate or stop enrollment at current dose level[b] |
| 9[c] | ≤ 2 | Consider re-escalate dose level or initiate part 2[b] |
| 9 [c] | ≥ 3 | De-escalate or stop enrollment at current dose level[c] |
| 10[c] | ≤ 2 | Consider re-escalate dose level or initiate part 2[b] |

DLT = dose-limiting toxicity

[a] Subject is evaluable if subject experiences a DLT or completes 21 days on treatment and receives  80% of planned dose of sotorasib.

[b] De-escalate guideline applies only when enrollment is allowed to a lower dose level. Re-escalation to the next higher Dose Level may be allowed, as appropriate.

[c] As appropriate to better understand safety profile for a dose level, the number of evaluable subjects may be expanded up to 10.

Objectives and Endpoints

| OBJECTIVES | ENDPOINTS |
|---|---|
| **PRIMARY** | |
| • To evaluate the safety and tolerability of sotorasib in adult subjects with Kirsten rat sarcoma (*KRAS*) *p.G12C* mutant advanced non-small cell lung cancer (NSCLC) with brain metastases | • Dose-limiting toxicities (DLTs), treatment-emergent adverse events, treatment-related adverse events, and changes in vital signs, electrocardiograms (ECGS), and clinical laboratory tests |
| **SECONDARY** | |
| • To evaluate the intracranial anti-tumor activity of sotorasib in adult subjects with *KRAS p.G12C* mutant advanced NSCLC with brain metastases | • Intracranial objective response rate (ORR) assessed per response assessment in neuro-oncology brain metastases (RANO-BM)<br>• Intracranial disease control rate (DCR), intracranial duration of response (DOR),duration of stable disease (DoSD), and time to intracranial radiation therapy assessed per RANO-BM<br>• Intracranial progression-free survival (PFS) assessed per RANO-BM |

(continued)

| SECONDARY | |
|---|---|
| • To evaluate anti-tumor activity of sotorasib adult subjects with *KRAS p.G12C* mutant advanced NSCLC | • Non-intracranial ORR, DOR, overall disease control rate, duration of stable disease, and PFS measured by computed tomography (CT) or magnetic resonance imaging (MRI) and assessed per response evaluation criteria in solid tumors version 1.1 (RECIST 1.1).<br>• Overall PFS assessed per RECIST 1.1 and Overall PFS assessed and RANO-BM<br>• Overall survival |
| • To explore PK/PD relationships for safety and/or efficacy endpoints | • Sotorasib pharmacokinetic (PK) parameters including, but not limited to, maximum observed plasma concentration ($C_{max}$), area under the plasma concentration-time curve (AUC) |

**Subject inclusion criteria include the following:**

Men or women at least 18 years old.

Pathologically documented, metastatic NSCLC with KRAS p.G12C mutation identified through molecular testing (performed according to in-country requirements), and with active brain metastases. Subjects must have received anti-PD-1 or anti programmed death-ligand 1 (PD L1) immunotherapy (unless contraindicated) AND/OR platinum-based combination chemotherapy AND targeted therapy (if actionable oncogenic driver mutations were identified -e.g.; EGFR, ALK, and ROS1), or if subject refused standard therapy. *KRAS p.G12C* mutation must be identified by an approved diagnostic device for detection of *KRAS p.G12C* in NSCLC or be performed in a Clinical Laboratory Improvement Amendments (CLIA) certified laboratory.

Metastatic brain disease meeting the following criteria:

- At least one measurable intracranial lesion > 10 mm;
- Subjects with largest measurable intracranial lesion > 5 mm but < 10 mm may be allowed to enroll upon agreement with investigator and Medical Monitor;
- CNS disease is asymptomatic on either a stable dose of corticosteroids or off corticosteroids (except for non-physiological doses) and off or on stable doses of non-enzyme-inducing antiepileptic drugs for at least 7 days. Corticosteroid doses or antiepileptic drugs must be discussed and agreed upon by the investigator;
- Lesions growing after whole-brain radiotherapy or resection may be allowed as target lesions upon agreement with investigator;
- Lesions growing after stereotactic radiosurgery may be allowed as target lesions only if radiation necrosis and pseudo-progression are determined to be unlikely upon agreement with investigator.

Subjects willing to provide archived tumor tissue samples (formalin fixed, paraffin embedded [FFPE] sample collected within 5 years) or willing to undergo pretreatment tumor biopsy. Subjects who do not have archived tissue available can be allowed to enroll without undergoing tumor biopsy upon agreement with investigator and the Medical Monitor if a tumor biopsy is not feasible.

Eastern Cooperative Oncology Group (ECOG) Performance Status of ≤ 1.

Life expectancy of > 3 months, in the opinion of the investigator.

Ability to take oral medications and willing to record daily adherence to investigational product.

Corrected QT interval (QTc) ≤ 470 msec for females and ≤ 450 msec for males (based on average of screening triplicates).

Adequate hematological laboratory assessments, as follows:

$$\text{Absolute neutrophil count (ANC)} \geq 1.5 \times 10^9/L$$

$$\text{Platelet count} \geq 100 \times 10^9/L$$

Hemoglobin ≥ 9 g/dL

Adequate renal laboratory assessments, as follows: Estimated glomerular filtration rate based on Modification of Diet in Renal Disease (MDRD) calculation ≥ 60 mL/min/1.73 $m^2$

Adequate hepatic laboratory assessments, as follows:

AST < 2.5 x ULN

(if liver metastases are present, ≤ 5 ULN)

ALT < 2.5 x ULN

(if liver metastases are present, ≤ 5 ULN)

Total bilirubin (BIL) < 1.5 x ULN

(<2.0 x ULN for subject with documented Gilbert's syndrome or < 3.0 x ULN for subject for whom the indirect BIL level suggests an extrahepatic source of elevation)

Adequate coagulation laboratory assessments, as follows: Prothrombin time (PT) or (activated) partial thromboplastin time (PTT or aPTT) < 1.5 x ULN, OR International Normalized Ratio (INR) < 1.5 or within target range if on prophylactic anticoagulation therapy.

**Exclusion criteria include the following:**

Spinal cord compression.

Patients with metastatic CNS disease other than brain metastasis as defined by the inclusion criteria.

Subject with known leptomeningeal disease.

History or presence of hematological malignancies unless curatively treated with no evidence of disease ≥ 2 years.

History of other malignancy within the past 2 years, with the following exceptions:

- Malignancy treated with curative intent and with no known active disease present for ≥ 2 years before enrollment and felt to be at low risk for recurrence by the treating physician
- Adequately treated non-melanoma skin cancer or lentigo maligna without evidence of disease
- Adequately treated cervical carcinoma in situ without evidence of disease
- Adequately treated breast ductal carcinoma in situ without evidence of disease
- Prostatic intraepithelial neoplasia without evidence of prostate cancer
- Adequately treated urothelial papillary non-invasive carcinoma or carcinoma in situ

Myocardial infarction within 6 months of study day 1, symptomatic congestive heart failure (New York Heart Association > class II), unstable angina, or cardiac arrhythmia requiring medication.

Gastrointestinal (GI) tract disease causing the inability to take oral medication, malabsorption syndrome, requirement for intravenous alimentation, uncontrolled inflammatory GI disease (e.g., Crohn's disease, ulcerative colitis).

Use of known cytochrome P450 (CYP) 3A4 sensitive substrates (with a narrow therapeutic window) or P-gp substrates, within 14 days or 5 half-lives of the drug or its major active metabolite, whichever is longer, prior to study day 1 that was not reviewed and approved by the principal investigator and the Medical Monitor.

Use of strong inducers of CYP3A4 (including herbal supplements such as St. John's wort) within 14 days or 5 half-lives (whichever is longer) prior to study day 1 that was not reviewed and approved by the principal investigator and the Amgen Medical Monitor.

Active infection requiring IV antibiotics within 1 weeks of study enrollment (day 1).

Evidence of hepatitis infection based on the following results and/or criteria: Positive Hepatitis B Surface Antigen (HepBsAg) (indicative of chronic Hepatitis B or recent acute hepatitis B); Negative HepBsAg with a positive for hepatitis B core antibody (Hepatitis B core antibody testing is not required for screening, however if this is done and is positive, then hepatitis B surface antibody [Anti-HBs] testing is necessary. Undetectable anti-HBs in this setting would suggest unclear and possible infection, and needs exclusion); Positive Hepatitis C virus antibody: Hepatitis C virus RNA by PCR is necessary. Detectable Hepatitis C virus RNA suggests chronic hepatitis C.

Known positive test for HIV.

Unresolved toxicities from prior anti-tumor therapy, defined as not having resolved to Common Terminology Criteria for Adverse Events (CTCAE) version 5.0 grade 0 or 1, or to levels dictated in the eligibility criteria with the exception of alopecia (grade 2 or 3 toxicities from prior anti-tumor therapy that are considered irreversible [defined as having been present and stable for > 6 months], such as ifosfamide-related proteinuria, may be allowed if they are not otherwise described in the exclusion criteria AND there is agreement to allow by both the investigator and sponsor.

Subject unable to receive gadolinium contrast for magnetic resonance imaging (MRI) scans. If MRI is contraindicated, computed tomography (CT) with and without contrast is acceptable.

Anti-tumor therapy (chemotherapy, antibody therapy, molecular targeted therapy, retinoid therapy, hormonal therapy [except for subjects with breast cancer], or investigational agent) within 15 days of study day 1; exception: subject who receive prior tyrosine kinase inhibitor monotherapy or conventional chemotherapy withn 14 to 28 days of study day 1 are eligible.

Therapeutic or palliative radiation therapy within 2 weeks of study day 1. Subjects must have recovered from all radiotherapy-related toxicity.

Currently enrolled in another investigational device or drug study, or less than 28 days since ending another investigational device or drug study(s), or receiving other investigational agent(s).

Previous treatment with a *KRAS p.G12C* inhibitor.

Major surgical procedures ≤ 28 days or non-study-related minor procedures ≤ 7 days prior to cycle 1 day 1. In all cases, the subjects must be sufficiently recovered and stable before treatment administration.

Currently receiving treatment in another investigational device or drug study, or less than 28 days since ending treatment on another investigational device or drug study(ies). Other investigational procedures while participating in this study are excluded.

Female subject is pregnant, breast feeding or planning to become pregnant or breastfeed during treatment and for an additional 7 days after receiving the last dose of sotorasib.

Female subjects of childbearing potential unwilling to use 1 highly effective method of contraception during treatment and for an additional 7 days after the last dose of sotorasib.

Female subjects of childbearing potential with a positive pregnancy test assessed at screening by a serum pregnancy test.

Male subjects with a female partner of childbearing potential who are unwilling to practice sexual abstinence (refrain from heterosexual intercourse) or use contraception during treatment and for an additional 7 days after the last dose of sotorasib.

Male subjects with a pregnant partner who are unwilling to practice abstinence or use contraception during treatment and for an additional 7 days after the last dose of sotorasib.

Male subjects unwilling to abstain from donating sperm during treatment with sotorasib and for an additional 7 days after the last dose of sotorasib.

Subject has known sensitivity to any of the products to be administered during dosing.

Subject will not be available for protocol-required study visits or procedures, to the best of the subject and investigator's knowledge.

Subject has any kind of disorder that, in the opinion of the investigator, may compromise the ability of the subject to give written informed consent and/or to comply with all required study procedures.

Excluded Treatments During Study Period

Excluded treatments/procedures for this study include:

Anti-tumor therapy such as chemotherapy, antibody therapy molecular targeted therapy

Known CYP3A4 sensitive substrates or P-gp substrates with narrow therapeutic indices that were not reviewed and approved by the principal investigator.

Strong CYP3A4 inducers that were not reviewed and approved by the principal investigator.

Systemic corticosteroids (prednisone ≥ 10 mg or equivalent) for reasons other than management of toxicities (e.g., immune-mediated adverse events), management of CNS disease symptoms, and non-physiological doses of steroids for subjects previously treated with steroids.

Concomitant Treatment

Breast cancer Resistant Protein (BCRP) substrates should be used with caution when co-administered with sotorasib, which may increase the circulating concentrations of BCRP substrates.

Concomitant administration of sotorasib with proton pump inhibitors (such as omeprazole, pantoprazole, etc), or H2 receptor antagonists (such as famotidine, ranitidine, etc.) is not recommended. If treatment with an acid-reducing agent is required, sotorasib should be taken 4 hours before or 10 hours after administration of a local antacid.

## Dose and Administration

Sotorasib will be administered orally daily (QD or BID) for a treatment cycle of 21 days with no planned off-treatment days. Sotorasib may be administered with or without food. Subject should take the sotorasib dose (all tablets at the same time) at approximately the same time every day. The sotorasib dose should also not be taken more than 2 hours earlier than the target time based on previous day's dose. The sotorasib dose should not be taken more than 6 hours after the dosing time. For BID dosing (every 12 hours), a subject should skip any missed sotorasib doses if 3 hours have passed from the scheduled time of dosing based on the previous day's dose, but instead wait and take the next dose as prescribed. If vomiting occurs after taking sotorasib, do not take an additional dose, but instead wait and take the next dose as prescribed.

Administration to patients who have difficulty swallowing solids: disperse tablets in 120 mL (4 ounces) of non-carbonated, room-temperature water without crushing. No other liquids should be used. Stir until tablets are dispersed into small pieces (the tablets will not completely dissolve) and drink immediately or within 2 hours. The appearance of the mixture may range from pale yellow to bright yellow. Swallow the tablet dispersion. Do not chew pieces of the tablet. Rinse the container with an additional 120 mL (4 ounces) of water and drink. If the mixture is not consumed immediately, stir the mixture again to ensure that tablets are dispersed.

## Dose Limiting Toxicities

The DLT window (i.e., DLT-evaluable period) will be the first 21 days of sotorasib. The grading of adverse events will be based on the guidelines provided in the CTCAE version 5.0. A subject will be DLT evaluable if the subject has completed the DLT window as described above and received ≥ 80% of the planned dose of sotorasib or experienced a DLT any time during the DLT window. A subject will not be DLT evaluable if he/she drops out before completion of the DLT evaluable period for reasons other than a DLT.

DLT is defined as any adverse event meeting the criteria listed below occurring during the DLT evaluation window and related to sotorasib:

- An adverse event that results in permanent discontinuation of any investigational product
- Grade ≥ 3 febrile neutropenia
- Grade 4 neutropenia of any duration
- Grade 3 neutropenia lasting > 7 days
- Grade 3 thrombocytopenia for > 7 days
- Grade 3 thrombocytopenia with grade > 2 bleeding
- Grade 4 thrombocytopenia
- Grade 4 anemia
- Grade ≥ 4, vomiting or diarrhea
- Grade 3 vomiting or Grade 3 diarrhea lasting more than 3 days despite optimal medical support
- Grade ≥ 3 nausea lasting 3 days or more despite optimal medical support
- Grade 3 ALT or AST elevations lasting more than 5 days and Grade ≥ 4 elevations of ALT or AST of any duration despite optimal medical management
- Grade ≥ 3 BIL elevation
- Any other grade > 3 adverse event with the following exceptions:

    - DLT Exemption: fatigue
    - DLT Exemption: Asymptomatic Grade 3 electrolyte abnormalities that last < 72 hours, are not clinically complicated, and resolve spontaneously or respond to medical interventions
    - DLT Exemption: Grade 3 amylase or lipase that is not associated with symptoms or clinical manifestations of pancreatitis
    - DLT Exemption: Other select lab abnormalities that do not appear to be clinically relevant or harmful to the patient and/or can be corrected with replacement or modifications (e.g., grade 3 lymphopenia, grade 3 hypoalbuminemia)

Any subject meeting the criteria for Hy's Law case (i.e., severe drug-induced liver injury [DILI]) will be considered a DLT. A Hy's Law case is defined as: AST or ALT values of ≥ 3 x ULN AND with serum TBL of > 2 x ULN without signs of cholestasis and with no other clear alternative reason to explain the observed liver related laboratory abnormalities

A recommendation to de-escalate to a lower dose cohort will only occur based on available study data through study day 28 for all subjects of a dose level and upon unanimous agreement of the DLRT voting members.

Dose reduction levels of sotorasib for toxicity management are provided below in the tables below.

Sotarasib dose reductions (Cohort A)

| Starting dose | Dose -1 | Dose -2 |
|---|---|---|
| 960 mg QD | 480 mg QD | 240 mg QD |
| QD = once daily | | |

Sotorasib dose reductions (Cohort B)

| Starting dose | Dose -1 | Dose -2 |
|---|---|---|
| 480 mg BID | 240 mg BID | 240 mg QD |
| BID - twice daily; QD = once daily | | |

Sotorasib will be discontinued, or dosage reduced, in the event of a toxicity that, in the opinion of the investigator, warrants the discontinuation, or dose reduction as indicated in the following Table. Dose reductions below 240 mg are not allowed. Subject who experiences an adverse event requiring dose reductions below 240 mg should be permanently discontinued from sotorasib treatment.

| | Recommended Action | |
|---|---|---|
| Toxicity | Hold Until: | Restart Dose:* |
| Grade ≥ 3 nausea, vomiting, or diarrhea lasting longer than 3 days despite optimal medical support | Recovery to grade 1 or less or to baseline grade | Resume dosing at 1 dose lower[a] |
| Any other drug-related toxicity ≥ grade 3[b] | Recovery to grade 1 or less or to baseline grade | Resume dosing at 1 dose lower[a] |
| Suspected interstitial lung disease (ILD)/pneumonitis of any grade | ILD/pneumonitis confirmed or excluded | If confirmed, permanently discontinue sotorasib. If excluded, restart at same dose if no other sotorasib dose modification guidelines are applicable. |
| *Subjects may be resumed at a dose lower than the recommended restarting dose after discussion with the medical monitor<br>[b]For subjects with hepatotoxicity, see below | | |

Hepatotoxicity Guidelines for Sotorasib: Guidelines for management and monitoring of subjects with increased AST, ALT, or alkaline phosphatase (ALP) are presented in the table below.

<table>
<tr><td colspan="4">• If the conditions for permanent discontinuation are met (below): Participant to be permanently discontinued<br>AST or ALT >3x ULN and INR > 1.5x ULN (for subjects not on anticoagulation therapy) in the presence of no important alternative causes for elevated AST/ALT values<br>OR<br>AST or ALT > 3x ULN and TBL > 2x ULN in the presence of no important alternative causes for elevated AST/ALT and/or TBL values<br>• If conditions are not met: Exclude other causes[a]<br>• Upon failure to identify any other causes and sotorasib relation to increase in LFTs cannot be excluded, proceed with guidelines below:</td></tr>
<tr><th>CTCAE Grade</th><th>Sotorasib Action</th><th>Medical Management</th><th>Monitoring and Follow-up</th></tr>
<tr><td>Grade 2 AST or ALT and ALP ≤ 8x ULN with no clinical symptoms consistent with hepatitis (right upper quadrant pain/tenderness, fever, nausea, vomiting, and jaundice)</td><td>Continue</td><td>Consider steroids[b]</td><td>• Closely monitor liver function tests</td></tr>
<tr><td rowspan="6">Grade 2 AST or ALT with symptoms<br><br>Or<br><br>Grade 3 or 4 AST or ALT<br><br>Or<br><br>8x ULN ALP[d]</td><td>First Occurrence</td><td rowspan="2">Initiate steroids[b]</td><td rowspan="2">• Closely monitor liver function tests<br>• Await resolution to baseline or grade 1 and resolution or improvement of hepatitis symptoms<br>• Restart at 1 dose level reduction[c, e]</td></tr>
<tr><td>Withhold</td></tr>
<tr><td>Second Occurrence</td><td rowspan="2">Initiate steroids[b]</td><td rowspan="2">• Closely monitor liver function tests<br>• Await resolution to baseline or grade 1 and resolution or improvement of hepatitis symptoms<br>• Resume at an additional 1 dose level reduction only with MEDICAL MONITOR approval[c, e]</td></tr>
<tr><td>Withhold</td></tr>
<tr><td>Third Occurrence</td><td colspan="2" rowspan="2">NOT APPLICABLE</td></tr>
<tr><td>Permanently discontinue Sotorasib</td></tr>
<tr><td colspan="4">ALP = alkaline phosphatase; ALT = alanine aminotransferase; AST = aspartate aminotransferase; CTCAE = Common Terminology Criteria for Adverse Events; INR = international normalized ratio; LFT = liver function test; TBL = total bilirubin; ULN = upper limit of normal<br>[a] If increase in AST/ALT is likely related to alternative agent, discontinue causative agent and await resolution to baseline or grade 1 prior to resuming sotorasib.<br>[b] For example: prednisone 1.0 to 2.0 mg/kg/day, dexamethasone equivalent, or methylprednisone or equivalent, followed by a taper. The taper may occur after restarting sotorasib. [c] Close monitoring at restart (e.g., daily LFTs x 2, then weekly x 4). Sotorasib dose may be increased after discussion with Medical Monitor.<br>[d] There is no limit to the number of sotorasib re-challenges for isolated alkaline phosphatase elevations that resolve to baseline or grade 1.<br>[e] Dose decrements below 240 mg are not allowable. Subjects may restart at same dose without dose reduction.</td></tr>
</table>

Hepatotoxicity Stopping Rules: Subjects with abnormal hepatic laboratory values (i.e., alkaline phosphatase (ALP), aspartate aminotransferase (AST), alanine aminotransferase (ALT), total bilirubin (TBL)) and/or international normalized ratio (INR) and/or signs/symptoms of hepatitis (as described below) may meet the criteria for withholding or

permanent discontinuation of sotorasib.

The following stopping and/or withholding rules apply to subjects for whom another cause of their changes in liver biomarkers (TBL, INR and transaminases) has not been identified. Important alternative causes for elevated AST/ALT and/or TBL values include, but are not limited to: Hepatobiliary tract disease; Viral hepatitis (e.g., hepatitis A/B/C/D/E, Epstein-Barr Virus, cytomegalovirus, herpes simplex virus, varicella, toxoplasmosis, and parvovirus); Right sided heart failure, hypotension or any cause of hypoxia to the liver causing ischemia; Exposure to hepatotoxic agents/drugs or hepatotoxins, including herbal and dietary supplements, plants and mushrooms; Heritable disorders causing impaired glucuronidation (e.g., Gilbert's syndrome, Crigler-Najjar syndrome) and drugs that inhibit bilirubin glucuronidation (e.g., indinavir, atazanavir); Alpha-one antitrypsin deficiency; Alcoholic hepatitis; Autoimmune hepatitis; Wilson's disease and hemochromatosis; Nonalcoholic fatty liver disease including steatohepatitis; and/or Non-hepatic causes (eg, rhabdomyolysis, hemolysis).

Rechallenge may be considered if an alternative cause for impaired liver tests (ALT, AST, ALP) and/or elevated TBL, is discovered and/or the laboratory abnormalities resolve to normal or baseline, as described in Table 10 below.

**Table 10**

| Analyte | Temporary Withholding | Permanent Discontinuation |
|---|---|---|
| TBL | > 3x ULN at any time | > 2x ULN<br>OR |
| INR | -- | > 1.5x (for subjects not on anticoagulation therapy) |
| | OR | AND |
| AST/ALT | > 5x ULN at any time<br>> 3x ULN with clinical signs or symptoms that are consistent with hepatitis (such as right upper quadrant pain/tenderness, fever, nausea, vomiting, and jaundice)<br>OR | In the presence of no important alternative causes for elevated AST/ALT and/or TBL values<br>> 3x ULN (when baseline was < ULN) |
| ALP | > 8x ULN at any time | -- |
| ALP = alkaline phosphatase; ALT = alanine aminotransferase; AST = aspartate aminotransferase; INR = international normalized ratio; TBL = total bilirubin; ULN = upper limit of normal | | |

## Biopsies

All subjects must have a mandatory tumor biopsy in any systemic lesion (core needle/fine needle aspirate for NSCLC), if medically feasible, obtained prior to treatment with sotorasib and once between week 2 to week 5 after starting treatment. If fresh biopsy is not medically feasible at screening, FFPE blocks or unstained slides of archived tumor tissue (collected within 5 years of enrollment) are sufficient.

An optional tumor biopsy in any systemic lesion, if medically feasible, should be performed at End of Treatment only if the subject has signed additional consent and has not started another anti-cancer therapy..

## Efficacy Assessments / Radiological Imaging Assessment

The extent of disease is evaluated by contrast-enhanced MRI/CT according to RECIST 1.1, as described below and RANO-BM. In order to reduce radiation exposure for subjects, the lowest dose possible should be utilized whenever possible.

The screening scans must be performed within 28 days prior to enrollment and are used as baseline. All subsequent scans are performed in the same manner as at screening, with the same contrast, preferably on the same scanner. Radiological assessment must include MRI/CT of the chest, abdomen and pelvis, as well as assessment of all other known sites of disease.

The same imaging modality, MRI field strength and intravenous and oral contrast agents should be used at screening should be used for all subsequent assessments. Liver specific MRI contrast agents should not be used. To reduce potential safety concerns, macrocyclic gadolinium contrast agents are recommended per National Health Institute guidelines, or follow local standards if more rigorous.

During treatment and follow-up, radiological imaging of the chest, abdomen, pelvis, as well as all other known sites of disease, will be performed independent of treatment cycle every 6 $\pm$ 1 weeks for the first 4 response assessments.

After 4 (6-week) response assessments, radiological imaging and tumor assessment will be performed every 12 ± 1 weeks. Imaging of the brain will be performed at 6 week intervals (± 1 week) throughout the duration of the study. Radiologic imaging and tumor assessment will be performed until disease progression, start of new anti-cancer treatment, death, withdrawal of consent or until end of study. Imaging may also be performed more frequently if clinically necessitated at the discretion of the managing physician. Radiographic response (complete response [CR], PR) requires confirmation by a repeat, consecutive scan at least 4 weeks after the first documentation of response and may be delayed until the next scheduled scan to avoid unnecessary procedures.

All subjects must have contrast-enhanced MRI of the brain performed within 28 days prior to first dose of sotorasib. Subsequently, brain scans may be performed at any time, if clinically indicated in the judgement of the managing physician. All brain scans on protocol are required to be MRI unless MRI is contraindicated, and then CT with and without contrast is acceptable.

Radiological imaging assessment during the end of treatment (EOT) visit should be performed only for subjects that discontinue treatment for a reason other than disease progression per RECIST 1.1 guidelines and/or RANO-BM guidelines.

Determination of disease response for clinical management of subjects is assessed at the clinical sites per RECIST 1.1 and RANO-BM criteria. Scans will be submitted to a central imaging core laboratory for archival and independent response assessment utilizing RECIST 1.1 and RANO-BM criteria. Exploratory imaging analyses may be performed centrally and may include tumor volumetrics, viable tumor measurements, tissue necrosis ratios, and lesion texture analysis (radiomics).

**Efficacy analyses**

The proportion of subjects with an intracranial objective response (per RANO-BM) and 95% CI will be tabulated by planned dose level. Disease control rate will be summarized similarly.

The proportion of subjects with an objective response (per RECIST 1.1) and 95% CI will be tabulated by planned dose level. Disease control rate will be summarized similarly.

For all subjects treated at the MTD and/or RP2D, Kaplan-Meier methods will be used to estimate the time to event curve, median time to event and percentiles with 95% CI for: 1) duration of response; 2) time to intercranial radiation therapy; 3) intracranial progression-free survival (PFS); 4) non intracranial PFS; 5) overall PFS, 6) duration of stable disease; and 7) OS, defined as the duration of time from the start of the study treatment until the event of death due to any cause (e.g., 1-year OS). Time to response will be summarized by the nonmissing sample size (n), mean, standard deviation, median, minimum, and maximum for responders.

For subjects who continue treatment post-progression, the first progression will be used for ORR/DOR and PFS analyses and subject's response post first progression will not be used to evaluate objective response endpoints.

**Response Evaluation Criteria in Solid Tumors Version 1.1 (RECIST 1.1)**

**Definitions**

Measurable Lesions

Measurable Tumor Lesions - Non-nodal lesions with clear borders that can be accurately measured in at least 1 dimension with longest diameter ≥ 10 mm in CT/MRI scan with slice thickness no greater than 5 mm. When slice thickness is greater than 5 mm, the minimum size of measurable lesion should be twice the slice thickness.

Nodal Lesions - Lymph nodes are to be considered pathologically enlarged and measurable, a lymph node must be ≥ 15 mm in short axis when assessed by CT/MRI (scan slice thickness recommended to be no greater than 5 mm). At baseline and in follow-up, only the short axis is measured and followed. Nodal size is normally reported as two dimensions in the axial plane. The smaller of these measures is the short axis (perpendicular to the longest axis).

Irradiated Lesions - Tumor lesions situated in a previously irradiated area, or in an area subjected to other loco-regional therapy, are not measurable unless there has been demonstrated progression in the lesion prior to enrollment.

Non-measurable Lesions: All other lesions, including small lesions (longest diameter < 10 mm or pathological lymph nodes with ≥ 10 mm but to < 15 mm short axis with CT scan slice thickness no greater than 5 mm) are considered non-measurable and characterized as non-target lesions.

Other examples of non-measurable lesions include: Lesions with prior local treatment: tumor lesions situated in a previously irradiated area, or an area subject to other loco-regional therapy, should not be considered measurable unless there has been demonstrated progression in the lesion; Biopsied lesions; Categorically, clusters of small lesions, bone lesions, and inflammatory breast disease are non-measurable.

Methods of Measurement

Measurement of Lesions - The longest diameter of selected lesions should be measured in the plane in which the images were acquired (axial plane). All measurements should be taken and recorded in metric notation. All baseline evaluations should be performed as closely as possible to the beginning of treatment and not more than 4 weeks before study Day 1.

Methods of Assessment - The same method of assessment and the same technique should be used to characterize each identified and reported lesion throughout the trial.

CT/ MRI - Contrast-enhanced CT or MRI should be used to assess all lesions. Optimal visualization and measurement of metastasis in solid tumors requires consistent administration (dose and rate) of IV contrast as well as timing of scanning. CT and MRI should be performed with ≤ 5 mm thick contiguous slices.

Baseline documentation of "Target" and "Non-target" lesions

Target Lesions - All measurable lesions up to a maximum of two (2) lesions per organ and five (5) lesions in total, representative of all involved organs should be identified as target lesions and recorded and measured at baseline.

Target lesions should be selected on the basis of their size (lesions with the longest diameter) and suitability for accurate repeated measurements.

Pathologic lymph nodes (with short axis ≥ 15 mm) may be identified as target lesions. All other pathological nodes (those with short axis ≥ 10 mm but < 15 mm) should be considered non-target lesions.

A sum of the diameters (longest for non-nodal lesions, short axis for nodal lesions) for all target lesions are calculated and reported as the baseline sum of diameters. The baseline sum of diameters are used as reference by which to characterize objective tumor response.

Non-Target Lesions - All other lesions (or sites of disease) including pathological lymph nodes should be identified as non-target lesions and should also be recorded at baseline. Measurements of these lesions are not required, and these lesions should be followed as "present", "absent", or "unequivocal progression" throughout the study. In addition, it is possible to record multiple non-target lesions involving the same organ as a single item on the case report form (e.g., "multiple enlarged pelvic lymph nodes" or "multiple liver metastases").

**Response Criteria**

**Evaluation of Target Lesions**

| | |
|---|---|
| * Complete Response (CR): | Disappearance of all target lesions. Any pathological lymph nodes (whether target or non-target) must have reduction in short axis to < 10 mm. |
| * Partial Response (PR): | At least a 30% decrease in the sum of the diameters of target lesions, taking as reference the baseline sum of diameters. |
| * Progressive Disease (PD): | At least a relative 20% increase and an absolute increase of 5 mm in the sum of the diameters of target lesions, taking as reference the smallest sum on study, or the appearance of 1 or more new lesions. |
| * Stable Disease (SD) | Neither sufficient shrinkage to qualify for PR nor sufficient increase to qualify for PD, taking as reference the smallest sum of diameters since the treatment started. |

**Evaluation of Non-target Lesions**

| | |
|---|---|
| * Complete Response (CR): | Disappearance of all non-target lesions and normalization of tumor marker levels. All lymph nodes must be non-pathological in size (< 10 mm short axis). |
| * Incomplete Response/ Stable Disease (SD): | Persistence of one or more non-target lesion(s) or/and maintenance of tumor marker levels above the normal limits. |

(continued)

| | |
|---|---|
| * Progressive Disease (PD) | Unequivocal progression of existing non-target lesions and/or appearance of one or more new lesions.[1] |
| [1] To achieve "unequivocal progression" on the basis of the non-target disease, there must be an overall level of substantial worsening in non-target disease such that, even in presence of SD or PR in target disease, the overall tumor burden has increased sufficiently to merit discontinuation of therapy. A modest "increase" in the size of 1 or more non-target lesions is usually not sufficient to qualify for unequivocal progression status. | |

**Response Assessment in Neuro-Oncology Brain Metastases (RANO-BM) Criteria**

This study utilizes the Response Assessment in Neuro-oncology Brain Metastases (RANO-BM) (Lin et al, 2015) criteria for assessment of intracranial response. RANO-BM is an extension of the Response Evaluation Criteria in Solid Tumors (RECIST) 1.1 (Eisenhauer et al, 2009) and the Response Assessment in Neuro oncology (RANO) (Wen et al, 2010) response assessment criteria for high-grade gliomas.

Definitions:

Measurable central nervous system (CNS) disease is defined as a contrast-enhancing CNS lesion that can be accurately measured in at least one dimension, with a minimum size of 10 mm, and is visible on two or more axial slices that are preferably 5 mm or less apart with 0 mm skip (and ideally ≤ 1.5 mm apart with 0 mm skip).

- The diameter perpendicular to the longest diameter in the plane of measurement should be at least 5 mm for the CNS lesion to be considered measurable.
- When more than one measurable CNS lesion is present at baseline, all CNS lesions (up to a maximum of 5) should be recorded and measured at baseline.
- If CNS lesions that are ≥ 5 mm but < 10 mm are to be considered as measurable disease, CNS lesions should be measured with magnetic resonance imaging (MRI) imaging with 1.5 mm slice thickness or less. Any CNS lesions < 10 mm in the longest diameter should be regarded as unchanged from baseline unless there is a minimum 3 mm change in the measured longest diameter.

Non-measurable CNS lesions include all other CNS lesions, including lesions with longest dimension < 10 mm, lesions with borders that cannot be reproducibly measured, dural metastases, bony skull metastases, and cystic-only lesions.

**Methods of Measurement**

Measurement of lesions: The longest diameter of selected lesions should be measured in the place in which the images were acquired.
Methods of Assessment: The same method of assessment and the same technique should be used to characterize each identified and reported lesion at baseline and throughout the trial.
MRI: Gadolinium-enhanced MRI should be used to assess all CNS lesions. Computed tomography (CT) with and without contrast may be performed if MRI is contraindicated.

Response Assessment for CNS Target Lesions

| Response Assessment For CNS Target Lesions | |
|---|---|
| Complete response (CR) | Disappearance of all CNS target lesions sustained for at least 4 weeks; with no new lesions, no use of corticosteroids, and patient is stable or improved clinically. |
| Partial response (PR) | At least a 30% decrease in the sum longest diameter of CNS target lesions, taking as reference the baseline sum longest diameter sustained for at least 4 weeks; no new lesions; stable to decreased corticosteroid dose; stable or improved clinically. |

(continued)

| Response Assessment For CNS Target Lesions | |
|---|---|
| Stable disease (SD) | Neither sufficient shrinkage to qualify for partial response nor sufficient increase to qualify for progressive disease, taking as reference the smallest sum longest diameter while on study. |
| Progressive disease (PD) | At least a 20% increase in the sum longest diameter of CNS target lesions, taking as reference the smallest sum on study (this includes the baseline sum if that is the smallest on study). In addition to the relative increase of 20%, at least one lesion must increase by an absolute value of 5 mm or more to be considered progression. |

For lesions that coalesce, a plane between them may be maintained that would aid in obtaining the maximum longest diameter of each individual lesion. If the lesions have coalesced and are no longer separable, the vector of the longest diameter in this instance should be the maximum longest diameter in this instance should be the maximum longest diameter for the coalesced lesion.

New CNS lesions, which were not present on prior scans, should be unequivocal and not due to technical or slice variation.

Non-target lesions should be assessed at least qualitatively with each radiographic assessment.

| Response Assessment For Non-Target Lesions | |
|---|---|
| Complete response ( CR) | Disappearance of all enhancing CNS non-target lesions, no new CNS lesions. |
| Non-complete response or non-progressive disease | Persistence of one of more non-target CNS lesion(s). |
| Progressive disease (PD) | Any of the following: unequivocal progression of existing enhancing non-target CNS lesions, new lesion(s), or unequivocal progression of existing tumor-related non-enhancing (t2/flair) CNS lesions. |

CNS and non-CNS compartments will be assessed separately as follows:

| **CNS (RANO-BM)** | **Non-CNS (RECIST 1.1)** | **Response** |
|---|---|---|
| CR, PR, or SD | CR, PR, or SD | CNS and non-CNS CR, PR, or SD, respectively |
| CR, PR, or SD | PD | CNS CR, PR, or SD respectively; non-CNS PD |
| PD | CR, PR, or SD | CNS PD; non-CNS CR, PR, or SD, respectively |
| PD | PD | CNS and non-CNS PD |

For assessment of overall progression free survival, if progression occurs in either or both compartments, the criterial for progression-free survival will have been met. Subjects who develop isolated CNS progression may be eligible to remain on study after local therapy (e.g., whole-brain radiotherapy, stereotactic radiosurgery, or surgery).

**Evaluation of Overall Response**

The best overall response is the best response recorded from the start of the study treatment until the end of treatment or disease progression/recurrence (taking as reference for PD the smallest measurements recorded since the treatment started).

In general, the subject's best response assignment depends on the findings of both target and non-target disease and also take into consideration the appearance of new lesions.

**Time Point response: Subjects with Target (+/- Non-target) Disease**

| Target Lesions | Non-target Lesions | New Lesions | Overall Response |
|---|---|---|---|
| CR | CR | No | CR |
| CR | Non-CR/non-PD | No | PR |
| CR | Not evaluated | No | PR |
| PR | Non-PD or not all evaluated | No | PR |
| SD | Non-PD or not all evaluated | No | SD |
| Not all evaluated | Non-PD | No | NE |
| PD | Any | Yes or No | PD |
| Any | PD | Yes or No | PD |
| NE = Not evaluable | | | |

**Time Point Response: Subjects with Non-Target Disease Only**

| Non-Target Lesions | New Lesions | Overall Response |
|---|---|---|
| CR | No | CR |
| Non-CR/non-PD | No | Non-CR/non-PD[1] |
| Not all evaluated | No | NE |
| Unequivocal PD | Yes or No | PD |
| Any | Yes | PD |
| [1] "Non-CR/non-PD" is preferred over "SD" for non-target disease since SD is increasingly used as endpoint for assessment of efficacy in some trials so as to assign this category when no lesions can be measured is not advised. | | |

**Overall Response: Confirmation of Complete Response (CR) and Partial Response (PR) required**

| Overall Response First Time Point | Overall Response Second Time Point | Best Overall Response |
|---|---|---|
| CR | CR | CR |
| CR | PR | SD, PD, or PR[1] |
| CR | SD | SD provided minimum criteria for SD duration met, otherwise, PD |
| CR | PD | SD provided minimum criteria for SD duration met, otherwise, PD |
| CR | NE | SD provided minimum criteria for SD duration met, otherwise, NE |
| PR | CR | PR |
| PR | PR | PR |
| PR | SD | SD |
| PR | PD | SD provided minimum criteria for SD duration met, otherwise, PD |
| PR | NE | SD provided minimum criteria for SD duration met, otherwise, NE |
| NE | NE | NE |
| [1] If a CR is truly met at first time point, then any disease at a subsequent time point, even if disease meeting PR criteria relative to baseline, makes the disease PD at that point (since disease must have reappeared after CR). | | |

Best response would depend upon whether minimum duration for SD was met. However, sometimes "CR" may be claimed when subsequent scans suggest small lesions were likely still present and in fact the subject had PR, not CR at

the first time point. Under these circumstances, the original CR should be changed to PR and the best response is PR.

**Special Notes on Response Assessment**

Nodal lesions - Lymph nodes identified as target lesions should always have the actual short axis measurement recorded, even if the nodes regress to below 10 mm on study. In order to qualify for CR, each node must achieve a short axis < 10 mm, NOT total disappearance. Nodal target lesion short axis measurements are added together with target lesion' longest diameter measurements to create the sum of target lesion diameters for a particular assessment (time point).

Target lesions that become "too small to measure" - While on study, all lesions (nodal and non-nodal) recorded at baseline should have their measurements recorded at each subsequent evaluation. If a lesion becomes less than 5 mm, the accuracy of the measurement becomes reduced. Therefore, lesions less than 5 mm are considered as being "too small to measure", and are not measured. With this designation, they are assigned a default measurement of 5mm. No lesion measurement less than 5mm should be recorded, unless a lesion totally disappears and "0" can be recorded for the measurement.

New lesions - The term "new lesion" always refers to the presence of a new finding that is definitely tumor. New findings that only may be tumor, but may be benign (infection, inflammation, etc.) are not selected as new lesions, until that time when the review is certain they represent tumor.

If a new lesion is equivocal, for example because of its small size, continued therapy and follow-up evaluation will clarify if it represents truly new disease. If repeat scans confirm there is definitely a new lesion, then progression should be declared using the date of the initial scan.

A lesion identified on a follow-up study in an anatomical location that was not scanned at baseline is considered a new lesion and will indicate disease progression, regardless of any response that may be seen in target or non-target lesions present from baseline.

Subjects with a global deterioration of health status requiring discontinuation of treatment without objective evidence of disease progression at that time should be classified as having "symptomatic deterioration." Every effort should be made to document the objective progression with an additional imaging assessment even after discontinuation of treatment.

In some circumstances it may be difficult to distinguish residual disease from scar or normal tissue. When the evaluation of complete response (CR) depends on this determination, it is recommended that the residual lesion be further investigated by fluorodeoxyglucose-positron emission tomography (FDG-PET) or PET/computed tomography (PET/CT), or possibly fine needle aspirate/biopsy, to confirm the CR status.

**Confirmation Measurement / Duration of Response**

Response Confirmation - In non-randomized trials where response is the primary endpoint, confirmation of PR and CR is required to ensure responses identified are not the result of measurement error.

Duration of overall response - The duration of overall response is measured from the time measurement criteria are first met for CR/PR (whichever is first recorded) until the first date the recurrent or progressive disease is objectively documented or death, whichever is earlier.

Duration of Stable Disease - SD is measured from the start of the treatment until the criteria for disease progression are met, taking as reference the smallest measurements recorded since the treatment started, or death, whichever is earlier.

ECOG Performance and NYHA Classification

| ECOG Performance Status Scale | |
|---|---|
| Grade | Descriptions |
| 0 | Fully active, able to carry on all pre-disease performance without restriction. |
| 1 | Restricted in physically strenuous activity, but ambulatory and able to carry out work of a light or sedentary nature (e.g., light housework, office work). |
| 2 | Ambulatory and capable of all self-care, but unable to carry out any work activities. Up and about more than 50% of waking hours. |
| 3 | Capable of only limited self-care, confined to bed or chair more than 50% of waking hours. |

(continued)

| ECOG Performance Status Scale | |
|---|---|
| Grade | Descriptions |
| 4 | Completely disabled. Cannot carry on any self-care. Totally confined to bed or chair. |
| 5 | Dead. |
| Source: Oken et al, 1982; [0001] ECOG = Eastern Cooperative Oncology Group; | |

New York Heart Association Functional Classification

Class I No limitation of physical activity. Ordinary physical activity does not cause undue fatigue, palpitation or dyspnea.

Class II Slight limitation of physical activity. Comfortable at rest, but ordinary physical activity results in fatigue, palpitation or dyspnea.

Class III Marked limitation of physical activity. Comfortable at rest, but less than ordinary activity causes fatigue, palpitation or dyspnea.

Class IV Unable to carry out any physical activity without discomfort. Symptoms of cardiac insufficiency may be present even at rest. If any physical activity is undertaken, discomfort is increased.

Preliminary data:

As of May 2, 2022 (the "cut-off date"), 6 subjects with non-small cell lung cancer and active brain metastases have been enrolled in Cohort A (960 mg QD). The subjects were treated for up to 36 weeks. The treatment is well tolerated with one subject having grade 3 events related to sotorasib, including a dose-limiting toxicity of Grade 3 hypokalemia. As of the cut-off date, the efficacy of the 960 mg QD dose was evaluable in 6 subjects and all subjects were off treatment. The preliminary results (best objective response) are shown in the table below. Tumor shrinkage in intercranial lesions is shown in Figure 1 by subject. These preliminary results show that a dose of 960 mg QD results in shrinkage of intercranial lesions in 4 out of 6 subjects, while the extracranial response indicates that 5 out of 6 subjects show an SD or PR response for the primary cancer, i.e., non-small cell lung cancer.

| Subject | Intercranial | Extracranial |
|---|---|---|
| 1 | PD | SD |
| 2 | SD | SD |
| 3 | SD | SD |
| 4 | SD | PR (Confirmed) |
| 5 | NE | PD |
| 6 | SD | SD |
| NE = not evaluated | | |

**Example 2 - Pharmacokinetic Analysis of 960 mg, 360 mg, 180 mg, and 240 mg Sotorasib**

Preliminary pharmacokinetic (PK) data were available for subjects with advanced solid tumors with the specific *KRAS p.G12C* mutation, with doses ranging from 180 to 960 mg PO QD of sotorasib (AMG 510). Dose-related increases in exposure on day 1 from 180 to 960 mg PO QD were observed. Increases in exposure were less than dose-proportional on day 1. There was no accumulation with multiple PO QD dosing for 8 days. The change in exposure from 180 to 960 mg PO QD was less than dose-proportional on day 8. Rapid absorption was observed with $t_{max}$ between 1 to 2 hours after PO administration. Figure 2 shows the mean plasma concentration time profile after oral administration of 180, 360, 720, or 960 mg sotorasib on Day 1. Figure 3 shows the concentrations after once daily dosing for 8 days (Day 8). The table below provides the pharmacokinetic parameters, where $AUC_{0-24h}$ is the area under the concentration-time curve from time 0 to 24 hr postdose; $C_{max}$ is the maximum observed drug concentration during a dosing interval; $t_{1/2,z}$ is the terminal elimination half-life; $t_{max}$ is the time to reach $C_{max}$, Data reported are presented as geometric mean (arithmetic CV%) except $t_{max}$ and $t_{1/2}$, which are reported as a median (range) and arithmetic mean (SD), respectively. Values are reported to three significant figures, except CV% and $t_{max}$, which are reported to 0 decimal places and 2 significant figures, respectively.

Pharmacokinetic Parameter

| Dose | | N | $t_{max}$ (hr) | $C_{max}$ (μg/mL) | $AUC_{0-24h}$ (hr•μg/mL) | $t_{1/2,z}$ (hr) |
|---|---|---|---|---|---|---|
| 180 mg | | | | | | |
| | Day 1 | 6 | 1.0 (0.50-2.0) | 6.88 (51%) | 44.0 (56%) | 5.95 (1.08)[a] |
| | Day 8 | 6 | 0.75 (0.50-1.0) | 6.44 (67%) | 33.5 (85%) | 5.96 (2.76)[b] |
| 360 mg | | | | | | |
| | Day 1 | 25 | 1.0 (0.50-24) | 5.86 (68%) | 56.8 (84%) | 6.56 (1.81)[c] |
| | Day 8 | 25 | 1.0 (0.25-4.0) | 5.97 (46%) | 37.4 (50%) | 5.71 (1.59)[d] |
| 720 mg | | | | | | |
| Day 1 | 11 | 1.0 (0.50-4.0) | 7.57 (59%) | 64.0 (68%) | 7.06 (1.59)[e] | |
| Day 8 | 11 | 1.0 (0.50-4.0) | 5.45 (50%) | 43.9 (49%) | 5.06 (1.24)[f] | |
| 960 mg | | | | | | |
| Day 1 | 25 | 2.0 (0.25-6.0) | 8.33 (59%) | 68.0 (77%) | 6.00 (2.20)[g] | |
| Day 8 | 25 | 1.0 (0.50-24) | 4.91 (69%) | 32.7 (70%)[h] | 5.19 (1.12)[i] | |
| [a]N=5; [b]N=6; [c]N=17;[d]N=19; [e]N=8; [f]N=9;[g]N=18; [h]N=24; [i]N=16; | | | | | | |

**Example 3 - Contraindication with co-administration of sotorasib with acid-reducing agents under fasted conditions**

This Phase 1, open-label, fixed-sequence study enrolled 14 healthy subjects. Subjects received 960 mg sotorasib on Day 1, 40 mg omeprazole once daily on Days 4 to 8, and 40 mg omeprazole followed by 960 mg sotorasib on Day 9. All doses were administered under fasted conditions. Blood samples for sotorasib PK were collected predose and up to 48 hours post-sotorasib dose. Sotorasib plasma PK parameters were estimated using non-compartmental methods.

Coadministration of sotorasib with omeprazole delayed sotorasib time to maximal plasma concentration ($t_{max}$) by 0.75 hours. Mean terminal half-life ($t_{1/2}$) of sotorasib was similar following coadministration of sotorasib with omeprazole compared to administration of sotorasib alone. Geometric mean sotorasib $AUC_{inf}$ (area under the curve from time zero to infinity) and $C_{max}$ (maximal plasma concentration) following coadministration of sotorasib with omeprazole (17000 h*ng/mL and 3100 ng/mL, respectively) were lower compared to administration of sotorasib alone (29300 h*ng/mL and 7200 ng/mL, respectively). Sotorasib was safe and well tolerated when coadministered with 40mg omeprazole or administered alone to healthy subjects.

Results indicated that coadministration of sotorasib with omeprazole, in the fasted state, decreased sotorasib $AUC_{inf}$ by 42% and $C_{max}$ by 57% compared with administration of sotorasib alone.

**Example 4** - **Contraindication with co-administration of sotorasib with acid-reducing agents under fed conditions**

This was a phase 1, open-label, fixed sequence, crossover, single-center study to explore mitigation strategies to limit the impact of acid-reducing agents on the exposure of sotorasib. This study evaluated the PK of sotorasib administered alone and in combination with famotidine or omeprazole in healthy men and women (a total of 14 subjects) under fed conditions. Subjects received a single dose of sotorasib on day 1, an evening dose of famotidine on day 3 (10 hours prior to sotorasib administration), a single dose of sotorasib on day 4 followed by another dose of famotidine 2 hours later, daily doses of omeprazole on day 6 through day 10, and a single dose of both omeprazole and sotorasib on day 11. All sotorasib administrations occurred following consumption of a standard calorie moderate fat meal. Blood was collected at predetermined timepoints to characterize plasma concentrations of sotorasib. Safety and tolerability monitoring was performed throughout the study.

A total of 15 healthy subjects (1 woman and 13 men) were enrolled in the study. Thirteen out of the 14 subjects received all treatments and completed the study.

Geometric least-square mean ratios of sotorasib $AUC_{inf}$ and $C_{max}$ were 0.622 and 0.654, respectively when comparing sotorasib coadministered with famotidine and sotorasib alone under fed conditions. Geometric least-square mean ratios of sotorasib $AUC_{inf}$ and $C_{max}$ were 0.430 and 0.349, respectively, when comparing sotorasib coadministered

with omeprazole and sotorasib alone. Doses of 960 mg sotorasib were safe and well tolerated with coadmnistered with a single dose of 40 mg famotidine and following multiple daily dosing of 40 mg omeprazole under fed conditions to healthy subjects.

In summary, coadministration of a single dose of famotidine (H2 receptor antagonist) given 10 hours prior to and 2 hours after a single dose of sotorasib under fed conditions decreased sotorasib $C_{max}$ by 35% and AUC by 38%. In addition, co-administration of repeat doses of omeprazole (PPI) with a single dose of sotorasib decreased sotorasib $C_{max}$ by 65% and AUC by 57% under fed conditions.

**Example 5 - Contraindication with coadministration of sotorasib with strong CYP34A4 inducers**

This Phase 1, open-label, fixed-sequence study enrolled 14 healthy subjects. Each subject received 960 mg sotorasib on Days 1, 3 and 18, and 600 mg rifampin on Day 3 and Days 5 to 19. Blood samples for sotorasib PK were collected predose and up to 48 hours post-sotorasib dose. Sotorasib plasma PK parameters were estimated using non-compartmental methods.

Results:

Geometric mean sotorasib $AUC_{inf}$ (area under the curve from time zero to infinity) and $C_{max}$ (maximal plasma concentration) following coadministration of single dose of rifampin with sotorasib (19600 h*ng/mL and 5340 ng/mL, respectively), were similar to those of sotorasib alone (25600 h*ng/mL and 6350 ng/mL, respectively). Geometric mean sotorasib $AUC_{inf}$ and $C_{max}$ following coadministration of multiple doses of rifampin with sotorasib (12400 h*ng/mL and 4110 ng/mL, respectively), were lower compared to those of sotorasib alone (25600 h*ng/mL and 6350 ng/mL, respectively).

Sotorasib was safe and well tolerated when coadministered with 600 mg rifampin or administered alone to healthy subjects. Single dose of rifampin did not have a clinically meaningful effect on sotorasib PK indicating sotorasib is not a substrate of OATP1B1. Multiple doses of rifampin decreased sotorasib $AUC_{inf}$ by 51% and $C_{max}$ by 35%, indicating sotorasib is a CYP3A4 substrate, consistent with in *vitro* data.

**Example** 6 - **Contraindication with coadministration of sotorasib with CYP34A substrates**

This Phase 1, open-label, fixed-sequence study enrolled X subjects with previously untreated NSCLC who received a single, oral dose of 2 mg midazolam alone of day -1, 960 mg sotorasib orally on days 1 through 14, and a single oral dose of 2 mg midazolam at approximately the same time as an oral dose of 960 mg sotorasib on day 15. Blood samples for sotorasib PK were collected predose and up to 48 hours post-sotorasib dose. Sotorasib plasma PK parameters were estimated using non-compartmental methods.

Single dose plasma midazolam PK data were obtained from 5 subjects who received midazolam alone and midazolam coadministered with sotorasib following 14 days of multiple daily dosing of sotorasib. Results indicated that exposure to midazolam decreased when coadministered with sotorasib following multiple daily dosing with sotorasib. Coadministration of sotorasib with midazolam (a sensitive CYP3A4 substrate) decreased midazolam $C_{max}$ by 48% and AUV by 53%.

**Example 7 - Contraindication with coadministration of sotorasib and P-gp substrates**

This Phase 1, open-label, fixed-sequence study enrolled 14 healthy subjects. Each subject received 0.5 mg digoxin on Day 1 and 960 mg sotorasib followed by 0.5 mg digoxin on Day 7. Blood samples for digoxin PK were collected predose and up to 144 hours post-digoxin dose. Samples were measured using validated high-performance liquid chromatography tandem mass spectrometry methods. PK parameters were estimated using non-compartmental methods. Safety and tolerability were monitored throughout the study.

Digoxin median time to maximal plasma concentration ($t_{max}$) and mean terminal half-life ($t_{1/2}$) were similar following coadministration of digoxin with sotorasib compared to those of digoxin alone. Geometric mean digoxin $AUC_{inf}$ (area under the curve from time zero to infinity) following coadministration of digoxin with sotorasib (40.3 h*ng/mL) was similar to that of digoxin alone (33.2 h*ng/mL). Geometric mean digoxin $C_{max}$ (maximal plasma concentration) following coadministration of digoxin with sotorasib (3.64 ng/mL) was higher compared to that of digoxin alone (1.90 ng/mL). Single doses of 0.5 mg digoxin were safe and well tolerated when administered alone or coadministered with 960 mg sotorasib.

Results indicated that coadministration of digoxin with a single dose of sotorasib increased digoxin $AUC_{inf}$ and $C_{max}$ by approximately 21% and 91%, respectively, compared with digoxin alone.

**References:**

Albert et al., (2007) Nat. Methods, 4:903-905.
Alizadeh et al., (1996) Nat. Genet., 14:457-460.
Beers and Nederlof, (2006) Breast Cancer Res., 8(3):210.
Bertone et al., (2006) Genome Res., 16(2):271-281.
Canon et al., (2019) Nature, 575:217-223.
Chung et al., (2004) Genome Res., 14(1):188-196.
Cecil Textbook of Medicine, (1985) W.B. Saunders & Co. pp. 2317-2341.
Cully M, Downward J., (2008) Cell, 133:1292.
Dalma-Weiszhausz et al., (2006) Methods Enzymol., 410:3-28.
Eisenhauer et al., (2009) Eur. J. Cancer, 45:228-247.
Flockhart DA, Drug Interactions: Cytochrome P450 Drug Interaction Table. Indiana University School of Medicine (2007), www.drug-interactions.medicine.iu.edu, accessed May 2021.
Forshew et al., (2012) Sci Transl Med.,; 4:136ra68
Haber and Velculescu, 2014 Cancer Discov., 4:650-61
Hong et al., (2020), N. Engl. J. Med., 383, 1207.
Hughes et al., (2001) Nat. Biotechnol., 19(4):342-347.
Irizarry, (2003) Nucleic Acids Res., 31:e15.
Janes et al., (2018) Cell, 172(3):578-589.
Jasmine et al., (2012) PLoS One, 7(2):e31968.
Kim et al., (2006) Carcinogenesis, 27(3):392-404.
Kinde et al., (2011) Proc Natl Acad Sci USA, 108:9530-5.
Kumar et al., (2012) J. Pharm. Bioallied Sci., 4(1):21-26.
Laere et al., (2009) Methods Mol. Biol., 512:71-98.
Lanman et al., (2020) J. Med. Chem., 63:52.
Lin et al., (2015) Lancet Oncol., 16:e270-278.
Lin et al., (2010) BMC Genomics, 11:712.
Liu et al., (2017) Biosens Bioelectron., 92:596-601.
Lodes et al., (2009) PLoS One, 4(7):e6229.
Mackay et al., (2003) Oncogene, 22:2680-2688.
Mao et al., (2007) Curr. Genomics, 8(4):219-228.
McDonald et al., (2017) Cell, 170(3):577-592.
Michels et al., (2007) Genet. Med., 9:574-584.
Mockler and Ecker, (2005) Genomics 85(1):1-15.
National Cancer Institute Common Terminology Criteria for Adverse Events v5.0 (NCI CTCAE).
Offin et al., (2019) Cancer, 125:4380-4387.
Ostrem et al., (2013) Nature, 503:548-551.
Ostrem and Shokat, (2016) Nature Rev Drug Discov.,15(11):771-785.
Patricelli et al., (2016) Cancer Discovery, 6:316-329.
Pinkel et al., (2005) Nat. Genetics, 37:S11-S17.
Simanshu et al., (2017) Cell, 170:17-33.
Thomas et al., (2005) Genome Res., 15(12):1831-1837.
Thompson et al., (2012) PLoS ONE, 7:e31597.
Villano et al., (2015) Neuro Oncol., 17:122-128.
Wang et al., (2012) Cancer Genet., 205(7-8):341-55,
Wei et al., (2008) Nucleic Acids Res., 36(9):2926-2938.
Wen et al., (2010) J Clin Oncol., 28:1963-72.
Xie et al., (2017) Front Pharmacol., 8:823.
Zubrod et al., (1960) J Chronic Disease, 11:7-33.
the cancer.

## Claims

1. A compound for use in treating cancer comprising a *KRAS G12C* mutation in a patient with active brain metastases,

wherein the compound is sotorasib and wherein sotorasib is administered orally.

2. The compound for use of claim 1, wherein the use comprises administering 960 mg of the compound to the patient once daily.

3. The compound for use of claim 1, wherein the use comprises administering 480 mg of the compound to the patient once daily.

4. The compound for use of claim 1, wherein the use comprises administering 240 mg of the compound to the patient once daily.

5. The compound for use of any one of claims 1-4, wherein the patient has an intracranial lesion that is greater than 5 mm.

6. The compound for use of claim 5, wherein the patient has an intracranial lesion that is greater than 10 mm.

7. The compound for use of any one of claims 1-4, wherein the cancer is non-small cell lung cancer, small bowel cancer, appendiceal cancer, colorectal cancer, cancer of unknown primary, endometrial cancer, mixed cancer types, pancreatic cancer, hepatobiliary cancer, small cell lung cancer, cervical cancer, germ cell cancer, ovarian cancer, gastrointestinal neuroendocrine cancer, bladder cancer, myelodysplastic/myeloproliferative neoplasms, head and neck cancer, esophagogastric cancer, soft tissue sarcoma, mesothelioma, thyroid cancer, leukemia, or melanoma.

8. The compound for use of any one of claims 1-6, wherein the cancer is non-small cell lung cancer.

9. The compound for use of any one of claims 1-6, wherein the cancer is colorectal cancer.

10. The compound for use of any one of claims 1-6, wherein the cancer is pancreatic cancer.

11. The compound for use of any one of claims 1-10, wherein the patient, prior to start of sotorasib therapy, had undergone at least one other systemic cancer therapy.

12. The compound for use of claim 11, wherein the patient had undergone at least two other systemic cancer therapies.

13. The compound for use of claim 11 or claim 12, wherein at least one systemic cancer therapy is selected from anti-PD-1 immunotherapy, anti-PD-L1 immunotherapy, and platinum-based chemotherapy.

14. The compound for use of claim 11, wherein the patient has previously undergone (i) an anti-PD1 therapy or anti-PD-L1 therapy, unless contraindicated, or (ii) a platinum-based chemotherapy, and (iii) an EGFR, ALK or ROS1 targeted therapy if the cancer also exhibited a mutation in EGFR, ALK, or ROS1.

15. The compound for use of claim 12, wherein the patient has previously undergone (i) an anti-PD1 therapy or anti-PD-L1 therapy, unless contraindicated, and (ii) a platinum-based chemotherapy, and (iii) an EGFR, ALK or ROS1 targeted therapy if the cancer also exhibited a mutation in EGFR, ALK, or ROS1.

16. The compound for use of any one of claims 1-15, wherein the patient exhibits an Eastern Cooperative Oncology Group (ECOG) performance status of 0 or 1.

17. The compound for use of any one of claims 1-16, wherein the cancer exhibits a PD-L1 tumor proportion score (TPS) of 1-49%.

18. The compound for use of any one of claims 1-16, wherein the cancer exhibits a PD-L1 tumor proportion score (TPS) of less than 1%.

19. The compound for use of any one of claims 1-16, wherein the cancer exhibits a PD-L1 tumor proportion score (TPS) of 50-100%.

20. The compound for use of any one of claims 1-19, wherein the cancer further comprises a *STK11* mutation.

21. The compound for use of any one of claims 1-19, wherein the cancer further comprises a *KEAP1* mutation.

22. The compound for use of any one of claims 1-19 and 21, wherein the cancer further comprises a *STK11* wild type.

23. The compound for use of any one of claims 1-20, wherein the cancer further comprises a *KEAP1* wild type.

## Patentansprüche

1. Eine Verbindung zur Verwendung bei der Behandlung einer Krebserkrankung, die eine KRAS-G12C-Mutation beinhaltet, bei einem Patienten mit aktiven Hirnmetastasen, wobei die Verbindung Sotorasib ist und wobei Sotorasib oral verabreicht wird.

2. Verbindung zur Verwendung gemäß Anspruch 1, wobei die Verwendung das einmal tägliche Verabreichen von 960 mg der Verbindung an den Patienten beinhaltet.

3. Verbindung zur Verwendung gemäß Anspruch 1, wobei die Verwendung das einmal tägliche Verabreichen von 480 mg der Verbindung an den Patienten beinhaltet.

4. Verbindung zur Verwendung gemäß Anspruch 1, wobei die Verwendung das einmal tägliche Verabreichen von 240 mg der Verbindung an den Patienten beinhaltet.

5. Verbindung zur Verwendung gemäß einem der Ansprüche 1-4, wobei der Patient eine intrakranielle Läsion aufweist, die größer als 5 mm ist.

6. Verbindung zur Verwendung gemäß Anspruch 5, wobei der Patient eine intrakranielle Läsion aufweist, die größer als 10 mm ist.

7. Verbindung zur Verwendung gemäß einem der Ansprüche 1-4, wobei die Krebserkrankung nichtkleinzelliger Lungenkrebs, Dünndarmkrebs, Appendixkrebs, Kolorektalkrebs, Krebs mit unbekanntem Primärtumor, Endometriumkrebs, gemischte Krebstypen, Bauchspeicheldrüsenkrebs, hepatobiliärer Krebs, kleinzelliger Lungenkrebs, Zervixkrebs, Keimzellkrebs, Eierstockkrebs, neuroendokriner Gastrointestinalkrebs, Blasenkrebs, myelodysplastische/myeloproliferative Neoplasien, Kopf- und Hals-Krebs, gastroösophagealer Krebs, Weichteilsarkom, Mesotheliom, Schilddrüsenkrebs, Leukämie oder Melanom ist.

8. Verbindung zur Verwendung gemäß einem der Ansprüche 1-6, wobei die Krebserkrankung nichtkleinzelliger Lungenkrebs ist.

9. Verbindung zur Verwendung gemäß einem der Ansprüche 1-6, wobei die Krebserkrankung Kolorektalkrebs ist.

10. Verbindung zur Verwendung gemäß einem der Ansprüche 1-6, wobei die Krebserkrankung Bauchspeicheldrüsenkrebs ist.

11. Verbindung zur Verwendung gemäß einem der Ansprüche 1-10, wobei sich der Patient vor Beginn der Sotorasibtherapie mindestens einer anderen systemischen Krebstherapie unterzogen hatte.

12. Verbindung zur Verwendung gemäß Anspruch 11, wobei sich der Patient mindestens zwei anderen systemischen Krebstherapien unterzogen hatte.

13. Verbindung zur Verwendung gemäß Anspruch 11 oder Anspruch 12, wobei mindestens eine systemische Krebstherapie aus Anti-PD-1-Immuntherapie, Anti-PD-L1-Immuntherapie und platinbasierter Chemotherapie ausgewählt ist.

14. Verbindung zur Verwendung gemäß Anspruch 11, wobei sich der Patient zuvor (i) einer Anti-PD1-Therapie oder Anti-PD-L1-Therapie, sofern nicht kontraindiziert, oder (ii) einer platinbasierten Chemotherapie und (iii) einer auf EGFR, ALK oder ROS1 gerichteten Therapie, falls die Krebserkrankung auch eine Mutation in EGFR, ALK oder ROS1 zeigte, unterzogen hat.

15. Verbindung zur Verwendung gemäß Anspruch 12, wobei sich der Patient zuvor (i) einer Anti-PD1-Therapie oder Anti-PD-L1-Therapie, sofern nicht kontraindiziert, und (ii) einer platinbasierten Chemotherapie und (iii) einer auf EGFR,

ALK oder ROS1 gerichteten Therapie, falls die Krebserkrankung auch eine Mutation in EGFR, ALK oder ROS1 zeigte, unterzogen hat.

**16.** Verbindung zur Verwendung gemäß einem der Ansprüche 1-15, wobei der Patient einen Eastern-Cooperative-Oncology-Group(ECOG)-Leistungsstatus von 0 oder 1 zeigt.

**17.** Verbindung zur Verwendung gemäß einem der Ansprüche 1-16, wobei die Krebserkrankung einen PD-L1-Tumor-Proportion-Score (TPS) von 1-49 % zeigt.

**18.** Verbindung zur Verwendung gemäß einem der Ansprüche 1-16, wobei die Krebserkrankung einen PD-L1-Tumor-Proportion-Score (TPS) von weniger als 1 % zeigt.

**19.** Verbindung zur Verwendung gemäß einem der Ansprüche 1-16, wobei die Krebserkrankung einen PD-L1-Tumor-Proportion-Score (TPS) von als 50-100 % zeigt.

**20.** Verbindung zur Verwendung gemäß einem der Ansprüche 1-19, wobei die Krebserkrankung ferner eine *STK11*-Mutation beinhaltet.

**21.** Verbindung zur Verwendung gemäß einem der Ansprüche 1-19, wobei die Krebserkrankung ferner eine KEAP1-Mutation beinhaltet.

**22.** Verbindung zur Verwendung gemäß einem der Ansprüche 1-19 und 21, wobei die Krebserkrankung ferner einen STK11-Wildtyp beinhaltet.

**23.** Verbindung zur Verwendung gemäß einem der Ansprüche 1-20, wobei die Krebserkrankung ferner einen *KEAP1*-Wildtyp beinhaltet.

## Revendications

**1.** Un composé pour son utilisation dans le traitement d'un cancer comprenant une mutation de *KRAS G12C* chez un patient présentant des métastases cérébrales actives, dans lequel le composé est le sotorasib et dans lequel le sotorasib est administré par voie orale.

**2.** Le composé pour l'utilisation de la revendication 1, dans lequel l'utilisation comprend l'administration de 960 mg du composé au patient une fois par jour.

**3.** Le composé pour l'utilisation de la revendication 1, dans lequel l'utilisation comprend l'administration de 480 mg du composé au patient une fois par jour.

**4.** Le composé pour l'utilisation de la revendication 1, dans lequel l'utilisation comprend l'administration de 240 mg du composé au patient une fois par jour.

**5.** Le composé pour l'utilisation de l'une quelconque des revendications 1 à 4, dans lequel le patient a une lésion intracrânienne qui est de plus de 5 mm.

**6.** Le composé pour l'utilisation de la revendication 5, dans lequel le patient a une lésion intracrânienne qui est de plus de 10 mm.

**7.** Le composé pour l'utilisation de l'une quelconque des revendications 1 à 4, dans lequel le cancer est un cancer du poumon non à petites cellules, un cancer de l'intestin grêle, un cancer de l'appendice, un cancer colorectal, un cancer primitif inconnu, un cancer de l'endomètre, des types de cancers mixtes, un cancer du pancréas, un cancer hépatobiliaire, un cancer du poumon à petites cellules, un cancer du col de l'utérus, un cancer des cellules germinales, un cancer de l'ovaire, un cancer neuroendocrinien gastrointestinal, un cancer de la vessie, des néoplasmes myélodysplasiques/myéloprolifératifs, un cancer de la tête et du cou, un cancer oesophagogastrique, un sarcome des tissus mous, un mésothéliome, un cancer de la thyroïde, une leucémie, ou un mélanome.

**8.** Le composé pour l'utilisation de l'une quelconque des revendications 1 à 6, dans lequel le cancer est un cancer du

poumon non à petites cellules.

9. Le composé pour l'utilisation de l'une quelconque des revendications 1 à 6, dans lequel le cancer est un cancer colorectal.

10. Le composé pour l'utilisation de l'une quelconque des revendications 1 à 6, dans lequel le cancer est un cancer du pancréas.

11. Le composé pour une utilisation de l'une quelconque des revendications 1 à 10, dans lequel le patient, avant de commencer une thérapie par sotorasib, a suivi au moins une autre thérapie systémique contre le cancer.

12. Le composé pour l'utilisation de la revendication 11, dans lequel le patient a suivi au moins deux autres thérapies systémiques contre le cancer.

13. Le composé pour l'utilisation de la revendication 11 ou de la revendication 12, dans lequel au moins une thérapie systémique contre le cancer est sélectionnée parmi l'immunothérapie anti-PD-1, l'immunothérapie anti-PD-L1, et la chimiothérapie à base de platine.

14. Le composé pour l'utilisation de la revendication 11, dans lequel le patient a précédemment suivi (i) une thérapie anti-PD1 ou une thérapie anti-PD-L1, sauf si cela était contre-indiqué, ou (ii) une chimiothérapie à base de platine, et (iii) une thérapie ciblée EGFR, ALK ou ROS1 si le cancer présentait également une mutation dans EGFR, ALK, ou ROS1.

15. Le composé pour l'utilisation de la revendication 12, dans lequel le patient a précédemment suivi (i) une thérapie anti-PD1 ou une thérapie anti-PD-L1, sauf si cela était contre-indiqué, et (ii) une chimiothérapie à base de platine, et (iii) une thérapie ciblée EGFR, ALK ou ROS1 si le cancer présentait également une mutation dans EGFR, ALK, ou ROS1.

16. Le composé pour l'utilisation de l'une quelconque des revendications 1 à 15, dans lequel le patient présente un indice de performance ECOG (*Eastern Cooperative Oncology Group*) de 0 ou 1.

17. Le composé pour l'utilisation de l'une quelconque des revendications 1 à 16, dans lequel le cancer présente un score de proportion tumorale (TPS) pour PD-L1 de 1 à 49 %.

18. Le composé pour l'utilisation de l'une quelconque des revendications 1 à 16, dans lequel le cancer présente un score de proportion tumorale (TPS) pour PD-L1 de moins de 1 %.

19. Le composé pour l'utilisation de l'une quelconque des revendications 1 à 16, dans lequel le cancer présente un score de proportion tumorale (TPS) pour PD-L1 de 50 à 100 %.

20. Le composé pour l'utilisation de l'une quelconque des revendications 1 à 19, dans lequel le cancer comprend en sus une mutation de *STK11.*

21. Le composé pour l'utilisation de l'une quelconque des revendications 1 à 19, dans lequel le cancer comprend en sus une mutation de *KEAP1.*

22. Le composé pour l'utilisation de l'une quelconque des revendications 1 à 19 et 21, dans lequel le cancer comprend en sus un type sauvage *de STK11.*

23. Le composé pour l'utilisation de l'une quelconque des revendications 1 à 20, dans lequel le cancer comprend en sus un type sauvage de *KEAP1.*

Figure 1

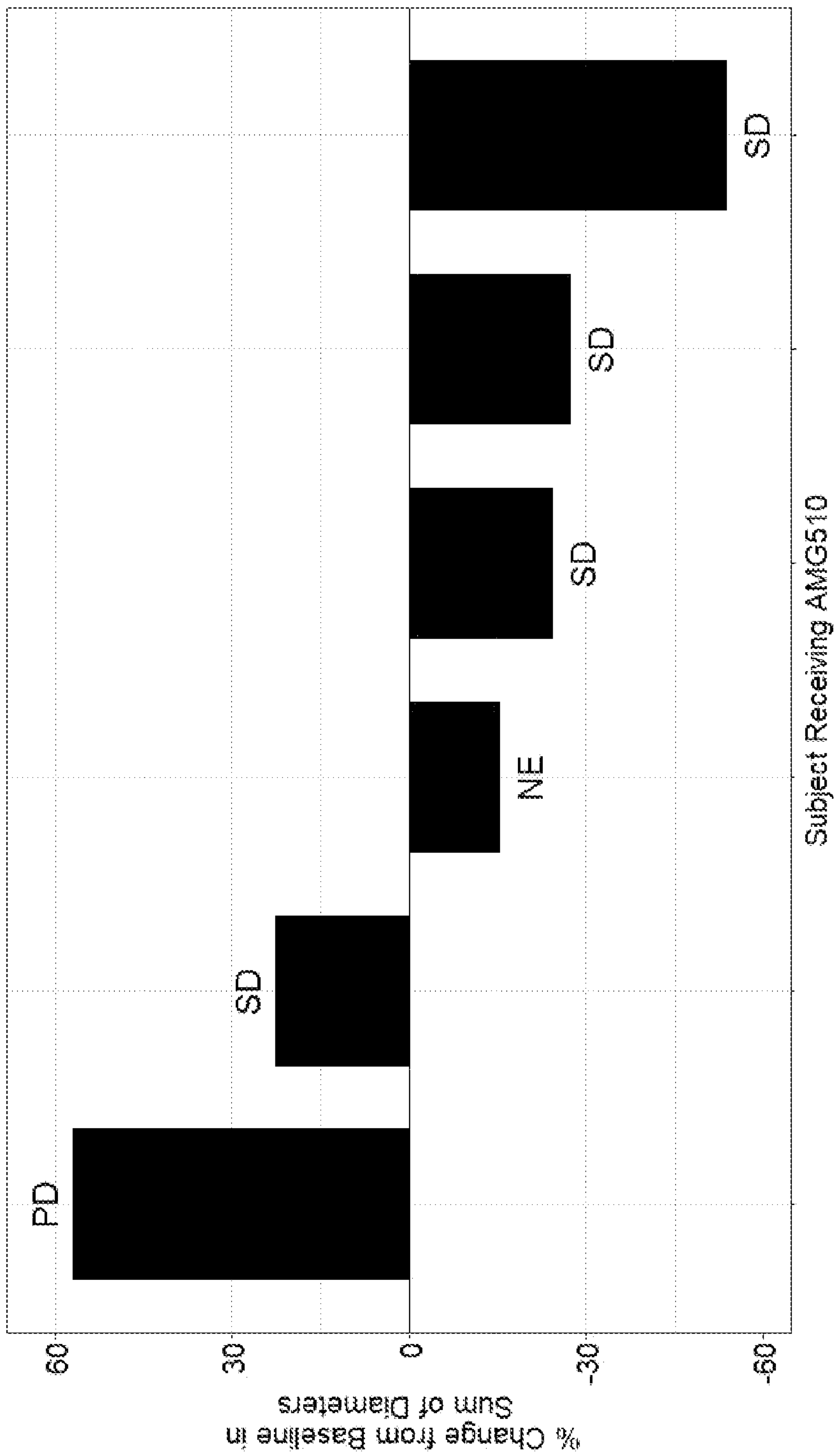
% Change from Baseline in
Sum of Diameters
60
30
0
-30
-60
PD
SD
NE
SD
SD
SD
Subject Receiving AMG510

Figure 2

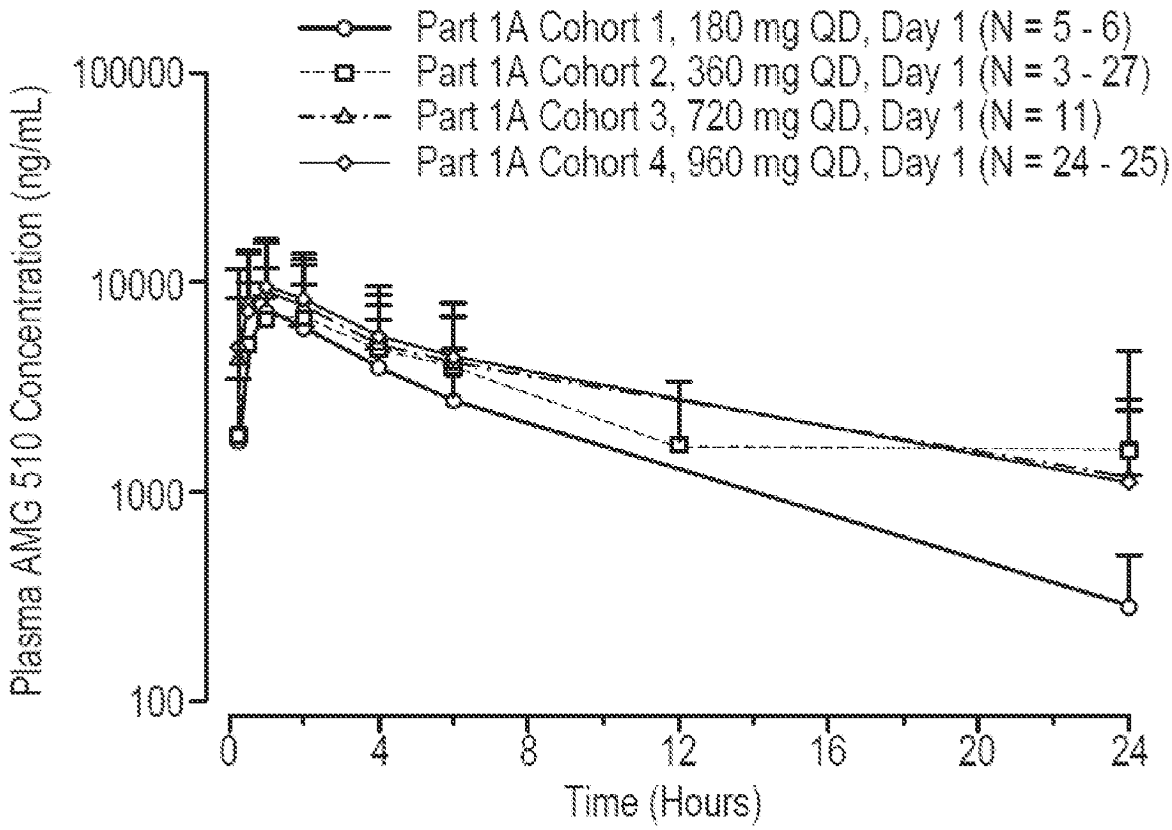
Part 1A Cohort 1, 180 mg QD, Day 1 (N = 5 - 6)
Part 1A Cohort 2, 360 mg QD, Day 1 (N = 3 - 27)
Part 1A Cohort 3, 720 mg QD, Day 1 (N = 11)
Part 1A Cohort 4, 960 mg QD, Day 1 (N = 24 - 25)
Plasma AMG 510 Concentration (ng/mL)
100000
10000
1000
100
0
4
8
12
16
20
24
Time (Hours)

Figure 3

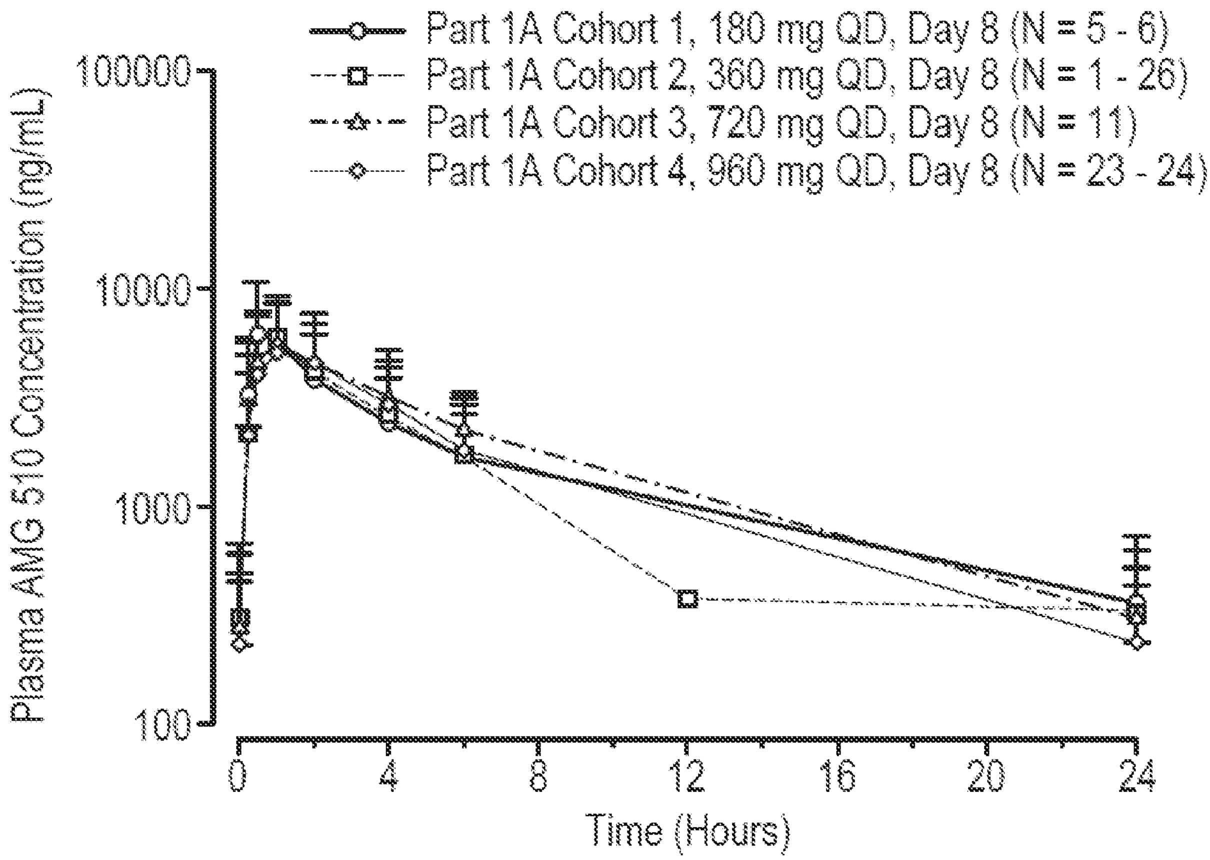
Part 1A Cohort 1, 180 mg QD, Day 8 (N = 5 - 6)
Part 1A Cohort 2, 360 mg QD, Day 8 (N = 1 - 26)
Part 1A Cohort 3, 720 mg QD, Day 8 (N = 11)
Part 1A Cohort 4, 960 mg QD, Day 8 (N = 23 - 24)
Plasma AMG 510 Concentration (ng/mL)
100000
10000
1000
100
0
4
8
12
16
20
24
Time (Hours)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63190061


### Non-patent literature cited in the description


- **HONG DAVID et al.** *N Engl J Med*, 24 September 2020, vol. 383 (13), 1207-1217
- National Cancer Institute Common Terminology Criteria for Adverse Events v5.0 (NCI CTCAE). National Cancer Institute, 27 November 2017
- Cecil Textbook of Medicine. W.B. Saunders & Co., 1985, 2317-2341
- **ALBERT et al.** *Nat. Methods*, 2007, vol. 4, 903-905
- **ALIZADEH et al.** *Nat. Genet.*, 1996, vol. 14, 457-460
- **BEERS** ; **NEDERLOF**. *Breast Cancer Res.*, 2006, vol. 8 (3), 210
- **BERTONE et al.** *Genome Res.*, 2006, vol. 16 (2), 271-281
- **CANON et al.** *Nature*, 2019, vol. 575, 217-223
- **CHUNG et al.** *Genome Res.*, 2004, vol. 14 (1), 188-196
- **CULLY M** ; **DOWNWARD J.** *Cell*, 2008, vol. 133, 1292
- **DALMA-WEISZHAUSZ et al.** *Methods Enzymol.*, 2006, vol. 410, 3-28
- **EISENHAUER et al.** *Eur. J. Cancer*, 2009, vol. 45, 228-247
- **FLOCKHART DA**. Drug Interactions: Cytochrome P450 Drug Interaction Table. Indiana University School of Medicine, 2007
- **FORSHEW et al.** *Sci Transl Med.*, 2012, vol. 4, 136-68
- **HABER** ; **VELCULESCU**. *Cancer Discov.*, 2014, vol. 4, 650-61
- **HONG et al.** *N. Engl. J. Med.*, 2020, vol. 383, 1207
- **HUGHES et al.** *Nat. Biotechnol.*, 2001, vol. 19 (4), 342-347
- **IRIZARRY**. *Nucleic Acids Res.*, 2003, vol. 31, e15
- **JANES et al.** *Cell*, 2018, vol. 172 (3), 578-589
- **JASMINE et al.** *PLoS One*, 2012, vol. 7 (2), e31968
- **KIM et al.** *Carcinogenesis*, 2006, vol. 27 (3), 392-404
- **KINDE et al.** *Proc Natl Acad Sci USA*, 2011, vol. 108, 9530-5
- **KUMAR et al.** *J. Pharm. Bioallied Sci.*, 2012, vol. 4 (1), 21-26
- **LAERE et al.** *Methods Mol. Biol.*, 2009, vol. 512, 71-98
- **LANMAN et al.** *J. Med. Chem.*, 2020, vol. 63, 52
- **LIN et al.** *Lancet Oncol.*, 2015, vol. 16, e270-278
- **LIN et al.** *BMC Genomics*, 2010, vol. 11, 712
- **LIU et al.** *Biosens Bioelectron.*, 2017, vol. 92, 596-601
- **LODES et al.** *PLoS One*, 2009, vol. 4 (7), e6229
- **MACKAY et al.** *Oncogene*, 2003, vol. 22, 2680-2688
- **MAO et al.** *Curr. Genomics*, 2007, vol. 8 (4), 219-228
- **MCDONALD et al.** *Cell*, 2017, vol. 170 (3), 577-592
- **MICHELS et al.** *Genet. Med.*, 2007, vol. 9, 574-584
- **MOCKLER** ; **ECKER**. *Genomics*, 2005, vol. 85 (1), 1-15
- **OFFIN et al.** *Cancer*, 2019, vol. 125, 4380-4387
- **OSTREM et al.** *Nature*, 2013, vol. 503, 548-551
- **OSTREM** ; **SHOKAT**. *Nature Rev Drug Discov.*, 2016, vol. 15 (11), 771-785
- **PATRICELLI et al.** *Cancer Discovery*, 2016, vol. 6, 316-329
- **PINKEL et al.** *Nat. Genetics*, 2005, vol. 37, S11-S17
- **SIMANSHU et al.** *Cell*, 2017, vol. 170, 17-33
- **THOMAS et al.** *Genome Res.*, 2005, vol. 15 (12), 1831-1837
- **THOMPSON et al.** *PLoS ONE*, 2012, vol. 7, e31597
- **VILLANO et al.** *Neuro Oncol.*, 2015, vol. 17, 122-128
- **WANG et al.** *Cancer Genet.*, 2012, vol. 205 (7-8), 341-55

- **WEI et al.** *Nucleic Acids Res.*, 2008, vol. 36 (9), 2926-2938
- **WEN et al.** *J Clin Oncol.*, 2010, vol. 28, 1963-72
- **XIE et al.** *Front Pharmacol.*, 2017, vol. 8, 823
- **ZUBROD et al.** *J Chronic Disease*, 1960, vol. 11, 7-33